# EUROPEAN PATENT SPECIFICATION

(11) **EP 1 737 354 B1**
(45) Date of publication and mention of the grant of the patent: **27.08.2014**
(21) Application number: 05740175.4
(22) Date of filing: 21.04.2005
(51) Int. Cl.: A61B 17/08

(54) **REMOTELY ANCHORED TISSUE FIXATION DEVICE**
ENTFERNT VERANKERTE GEWEBEFIXATIONSVORRICHTUNG
DISPOSITIF DE FIXATION D'UN TISSU ANCRE A DISTANCE

(30) Priority: 23.04.2004 US 831755; 02.11.2004 US 980494
(43) Date of publication of application: 03.01.2007
(73) Proprietor: MicroAire Surgical Instruments, LLC, Charlottesville, VA 22911 (US)
(72) Inventor: JACOBS, Daniel, Irvin, Palo Alto, CA 94306 (US); ELSON, Robert, J., Los Altos Hills, CA 94022 (US); DAVIS, Melissa, Palo Alto, California 94303 (US); MORRISS, John H., Palo Alto, California 94303 (US); SENATORI, Mark, San Francisco, CA 94121-3502 (US); LAMPS, Greg, Sunnyvale, CA 94086 (US)
(74) Representative: Howell, Matthew Dafydd
(86) International application number: PCT/US2005/013732
(87) International publication number: WO 2005/104962

(56) References cited:
- EP-A2- 1 356 778
- WO-A1-93/08727
- WO-A1-95/26170
- WO-A1-03/013368
- WO-A2-01/89392
- WO-A2-03/047416
- DE-A1- 3 631 762
- GB-A- 2 126 903
- US-A- 3 646 615
- US-A- 3 646 615
- US-A- 4 246 660
- US-A- 4 501 029
- US-A- 4 820 305
- US-A- 5 916 224
- US-A- 5 919 234
- US-A1- 2003 093 117
- US-B1- 6 241 747
- US-B2- 6 692 499

## Description

### FIELD OF THE INVENTION

This invention is in the field of surgery. More particularly, it relates to a tissue approximation device. By "approximation" we mean to include variously the specific movement of two regions of tissue towards each other, the movement of one or more selected tissue regions or areas, the maintenance and/or fixation of one or more selected tissue regions in a selected position, and the maintenance and/or fixation of a selected area of tissue against shape variation due to tissue "springiness." We will also refer to these functions as "stabilization" of a tissue region. For instance, the inventive device may be used to facilitate wound healing by holding soft tissue together under improved distribution of tension and with minimal disruption of the wound interface and its nutrient supplies. Generally, the device has multiple sites for grasping said tissue using tines or prongs or other generally sharp, projecting points, extending from and preferably affixed to a single, supportive backing. Various processes of using the inventive device are also a portion of the invention.

### BACKGROUND OF THE INVENTION

The inventive device is preferably used for the approximation, mobilization, or fixation of tissue. As noted above, these terms are meant variously to include the specific movement of two regions of tissue towards each other, the movement of one or more selected tissue regions or areas, the maintenance of one or more selected tissue regions in a selected position, and the maintenance of a selected area of tissue against shape variation due to tissue "springiness." Using our inventive device, a variety of approximation procedures may be achieved, variously from the movement of two tissue areas towards each other at a common wound margin to the maintenance of an area of tissue in a specific position during or after a surgical procedure, e.g. soft tissue in the middle and lower regions of the face or in the neck.

For instance, our inventive device allows healing of soft tissue due to its maintenance of tissue position. The surgically induced healing of soft tissue wounds involves two phases, the mechanical phase of wound closure followed by the biochemical phase which involves protein bridging and scarring. In the mechanical phase, the edges of soft tissue are held in contact by essentially two components: 1) The physical properties and device-tissue interactions of the materials holding the tissue edges in contact, e.g. sutures or staples; and 2) An early deposition of proteinaceous material that has adhesive characteristics, e.g. fibrin glue.

Only in the biochemical phase, which occurs after the mechanical phase, do tissue components replace the mechanical components adhering the displaced or wounded soft-tissue surfaces. During the biochemical phase, the inflammatory cascade generates signals which induce fibroblasts to migrate into the site or sites of wound healing and synthesize collagen fibers.

Collagen is the primary constituent of connective tissue and ultimately determines the pliability and tensile strength of the healing wound. Tensile strength is gradually recovered; 60% of ultimate wound strength is achieved after approximately 3 months. However, this process is successful only if the previous mechanical phase has proceeded normally.

The surgeon's goal is to optimize the strength and often the cosmetic appearance of a wound closure or tissue coaptation. For this to happen, tissue is mechanically approximated until the wound has healed enough to withstand stress without artificial support. Optimal healing requires the application of appropriate tissue tension on the closure to minimize or eliminate dead space but not create ischemia within the tissue. Both of these circumstances increase the risk of wound infection and wound dehiscence.

Although the biomaterial composition of sutures has progressed considerably, the sophistication of manual suture placement in wounds has advanced relatively little since the original use of fabrics several thousand years ago to tie wound edges together. The wide tolerance ranges for suture placement, tension, and configurations, both amongst different surgeons and for different implementations by the same surgeon, result in a significant component of sub-optimal technique. Yet, the technique used for wound closure forms the foundation for all subsequent events in the healing process. It is during this mechanical phase that tissue tension is high, edema and inflammation are intense, ischemia around the detached or wounded soft tissue is greatest, and that one can already observe the complication of optimal healing and fixation.

Soft tissue is well known for its inability to hold tension. Even when optimally placed, sutures gradually tear through soft tissue, producing gaps in wounds and possibly leading to the eventual failure or sub-optimization of wound healing. Furthermore, since sutures require the implementation of high levels of tension to counteract the forces acting to separate tissues, they may strangulate the blood supply of the tissues through which they are placed, thus inhibiting the delivery of nutrients and oxygen necessary for healing at and near the site of tissue fixation and repair.

There have been many attempts to construct wound closure devices that decrease closure time and improve cosmesis. U.S. Pat. Nos. 2,421,193 and 2,472,009 to Gardner; U.S. Pat. No. 4,430,998 to Harvey et al.; U.S. Pat. No. 4,535,772 to Sheehan; U.S. Pat. No. 4,865,026 to Barrett; U.S. Pat. No. 5,179,964 to Cook; and U.S. Pat. No. 5,531,760 to Alwafaie suggest such devices. However, these devices are not useful in surgical or deeper wounds. They only approximate the skin surface, joining skin edges variously through external approaches, using adhesives or nonabsorbable attachment points that penetrate tissue. The devices minimally improve the biomechanics of wound closure, and do not adequately approximate the deeper layers of the closure, i.e. fascia or dermis. Externally placed attachment points that puncture the skin lateral to the wound also interfere with long-term cosmesis and provide a possible conduit for infecting micro-organisms.

U.S. Pat. No. 5,176,692 to Wilk et al., discloses a device for hernia repair that utilizes mesh with pin-like projections to cover hernia defects. This device, however, is used in a laparoscopic hernia repair in conjunction with an inflatable balloon. Closure devices for deeper tissues are described in U.S. Pat. No. 4,610,250 to Green; U.S. Pat. No. 5,584,859 to Brozt et al.; and U.S. Pat. No. 4,259,959 to Walker. However, these devices either work in conjunction with sutures, are made of materials that do not suggest biodegradability, or are designed in such a way as not to impart uniform tension on the closure, increasing the risk of wound separation and failure of wound healing.

The present invention is a tissue approximation assembly comprising an implantable tissue approximation device and a delivery device according to appended claim 1. The device includes a plurality of attachment points, e.g. tines, prongs, or other generally sharp or blunt parts, connected to one or more backings that can be manipulated to close wounds, join soft tissue or bone, approximate regions of soft tissue or create anastomoses. This multi-point tension distribution system device may be placed with minimal tissue trauma. Approximation from the internal aspect of the wound minimizes the potential for dead space in the closure, thus decreasing the risk of sub-optimal healing. Moreover, because the device is absorbed, a second procedure is not typically needed to remove the device.

Thus, the present invention improves the mechanical phase of healing and tissue approximation by facilitating the coaptation of tissues prior to initiation of the biochemical phase of biological healing. Placement of the device maximizes the chance for a good cosmetic result and is not heavily dependent on surgeon skill.

A variation of the present invention is well suited for inferior orbital rim, craniofacial, and maxillofacial reconstructive procedures.

Current orbital rim, craniofacial, and maxillofacial reconstructive procedures have a number of problems to overcome. The problems to be overcome arise from elevating the soft tissue or skin off the bone repair site. Elevating the soft tissue is generally necessary to access and repair the bone site. Typically, the fractured bones are set using a fracture fixation device such as a biocompatible or biodegradable plate which is attached to the underlying fractured bones using screws.

After the bone site is repaired, however, the soft tissue which was elevated must be re- anchored. Failure to re-anchor the soft tissue results in undesirable sagging or drooping.

Conventional techniques to reduce the sagging and drooping of soft tissue in these regions utilize sutures. Sutures are typically attached to screws or anchors or the bone itself via a drill hole. The soft tissue is then attached to the suture. This conventional technique is undesirable for the reasons set forth above in connection with the use of sutures.

The present invention overcomes the above noted problems by providing the inventive features herein described. In particular, the present invention provides one or more attachment points to hang soft tissue in the orbital, craniofacial, and maxillofacial regions to prevent sagging without the use of sutures. Furthermore, use of the present invention provides a one- step procedure for orbital fracture fixation and tissue approximation or fixation.

From patent publication WO03/047416A2 there is known a fixation plate and techniques for fixation of a fracture in wrist surgery. However, in this plate attachment points are at or close to a longitudinal side edge.

From patent publication GB2126903A there is known a pronged plate for resetting fractured bones. However, in this plate prongs are located at a longitudinal side edge.

From patent publication US3646615A there is known a reinforcing element for muscles. However, in this reinforcing element there are teeth on the side edges.

From patent publication US4246660A there is known an artificial ligament. However, in this artificial ligament an elongated member does not have a plurality of through-holes.

From patent publication WO95/26170A1 there is known a microsurgical clamp. However, in this microsurgical clamp there is no backing having front and back sides and longitudinal sides.

From patent publication EP1356778A2 there is known a stabilizing support for opening- and closing-wedge osteotomies. However, this stabilizing support has no supportive backing.

From patent publication WO01/89392, there is known a tissue approximation device. However, there is no disclosure of any delivery means. The two-part form of independent claim 1 is based on this document.

Other advantages of the present invention will become apparent from the following disclosure.

### SUMMARY OF THE INVENTION

The present invention is an implantable tissue approximation assembly as claimed in claim 1.

Other objects and features of the present invention will become apparent by a review of the specification, claims and appended figures.

### BRIEF DESCRIPTION OF THE DRAWINGS

FIGS. 1A-1D are plan, perspective views of various devices.
FIGS. 2A-2E are side views of various attachment point shapes and orientations.
FIGS. 3A-3D and 3F-3G are side views of various attachment points.
FIG. 3E is a side view of a two-sided device.
FIG. 3H is a plan, reverse perspective view of nubs on the inferior surface of a device.
FIG. 4A is a side, cross-sectional view of attachment points that run through the width of a backing
FIG. 4B is a side view of attachment points on a strip of backing material.
FIG. 4C is a plan, perspective view of the points in 4B on a backing.
FIG. 4D is a plan, perspective view of attachment points on a solid backing.
FIG. 5A is a plan, perspective view of attachment points canted in one direction.
FIGS. 5B-5D are plan, perspective views of attachment points with various orientations on a backing.
FIG. 5E is a side view of attachment points becoming progressively shorter the closer they are to the center of the device.
FIG. 5F is a side view of attachment points becoming progressively shorter the farther they are from the center of the device.
FIGS. 6A-6B are schematic views of a skin wound and wound repair using the device.
FIG. 7 is a schematic view of an abdominal wound closure using devices.
FIGS. 8A-8B are schematic views of an abdominal hernia and hernia repair using the device.
FIGS. 8C-8D are side and schematic views, respectively, of a device with attachment points on the edges of the backing and a central area without attachment points.
FIGS. 9A-9B are schematic views of a ruptured tendon and tendon to bone repair using the device.
FIG. 10A is an axial view of a cross-section of a vessel repaired with the device.
FIGS. 10B-10C are side, schematic views of vessel free ends and a vascular anastomosis using the device.
FIGS. 11A and 11B-11C are schematic, side, and cross-sectional side views, respectively, of a transected tendon and a tendon to tendon repair using the device.
FIG. 11D is an axial, cross-sectional view of the tendon to tendon
FIG. 11E is a side view of a vascular anastomosis using the device on the external surface of a vessel.
FIGS. 11F-11G are side, schematic views, and FIG. 11H is an axial view of the ends of a tubular structure being joined by externally placing strips of a device on approximated tissue.
FIG. 11I is an axial view of a hinge in the backing of a device.
FIGS. 11J-11K are axial views of decreased backing material that are areas of enhanced device flexibility.
FIGS. 11L-11M are side views of a spring or coil-like device being used to approximate tissue.
FIG. 12A is a schematic view of the device being used in a brow-lift procedure.
FIG. 12B is a plan, perspective view of the device used in a brow-lift.
FIG. 13A is a front view of a variation of a device having an integral post or anchor used in a brow-lift.
FIGS. 13B-13C are a top view and a side view, respectively, of the device of FIG. 13A showing the attachment points and integral post.
FIG. 13D is a perspective view of the device of FIG. 13A.
FIG. 13E is a view of cross-section 13E-13E from FIG. 13B showing the cavities in the post.
FIGS. 14A-14D show a top view of a patient's cranium during insertion of the device of FIG. 13A.
FIG. 15 is a cross-sectional side view of the insertion and securing procedure of the device from FIG. 14C.
FIGS. 16A-16D are various views of an exemplary attachment point from FIG. 13A.
FIG. 17A is a view from perspective 17A-17A from FIG. 13C of the post having a partial collar.
FIG. 17B is a variation of FIG. 17A of the post having a full collar.
FIG. 17C is a variation of FIG. 17A of the post having several tabs.
FIGS. 18A-18C show back, front, and side views of a post variation missing a distal cavity.
FIG. 19A is a perspective view of the post from FIG. 18B showing the proximal cavity within the post.
FIG. 19B is a view of cross-section 19B-19B from FIG. 18B showing the proximal cavity.
FIG. 20 is a perspective view of a post variation having a beveled latching mechanism.
FIG. 21 is a perspective view of another post variation having an integral beveled latching mechanism.
FIG. 22A is a side view of a post variation having a rounded hook.
FIG. 22B is a side view of a post variation having an angled post.
FIG. 22C is a side view of the supportive backing defining a hole to receive a separate fastening device.
FIGS. 22D-22E are side views of a radially expandable post variation.
FIG. 23A is a cross-sectional view of a typical hole in a patient's cranium for receiving a post.
FIG. 23B is a cross-sectional view of an angled hole variation for receiving a post.
FIG. 23C is a cross-sectional view of a possible keyed hole variation for receiving a post.
FIGS. 24A-24C are top, side, and perspective views of an alternative variation of the device.
FIG. 24D is a view of cross-section 24D-24D from FIG. 24A.
FIGS. 25A-25C are top, side, and back views of another variation of the device which may receive separatable attachment points.
FIGS. 26A-26C are top, side, and back views of a variation of the device having dual tabs on the post.
FIGS. 27A-27C are top, side, and back views of a variation of the device having a latching mechanism on the post.
FIGS. 28A-28C are top, side, and perspective views of a variation of the device having another latching mechanism on the post.
FIG. 28D is a view of cross-section 28D-28D from FIG. 28A.
FIGS. 29A-29C are edge, back, and side views of a variation of the device having two adjacent posts.
FIGS. 30A-30C are edge, back, and side views of another variation of the device having two aligned posts.
FIG. 31A is a top view of a variation of the insertion tool showing the channel.
FIG. 31B is a view of cross-section 31B-31B from FIG. 31A showing an device and a side view of the support block.
FIG. 31C is a close-up view of the device and support block from FIG. 31B.
FIG. 31D is a perspective view from the bottom showing the insertion tool of FIG. 31A.
FIG. 31E is a perspective view from the top showing the insertion tool of FIG. 31A.
FIG. 32A is a top view of the insertion tool from FIG. 31A showing the block assembly.
FIG. 32B is a view of cross-section 32B-32B from FIG. 32A showing the device and a side view of the block assembly.
FIG. 32C is a close-up view of the device and block assembly from FIG. 32B.
FIG. 32D is a perspective view from the bottom showing the insertion tool of FIG. 32A.
FIG. 32E is a perspective view from the top showing the insertion tool of FIG. 32A.
FIGS. 33A-33D are front views of another device.
FIG. 33E is an exemplary device.
FIG. 34A is another variation of an exemplary device.
FIG. 34B is a side view of the device shown in FIG. 34A.
FIG. 35A is a front view of another variation of a device.
FIG. 35B is a side view of the device shown in FIG. 35A.
FIG. 35C is a side view of another variation of a device.
FIGS. 36A-36C are front, top, and side views of another variation of a device.
FIGS. 37A-37D are illustrations of another variation.
FIGS. 38A and 38B are illustrations of a variation in which a backing is adjustably positioned on a connecting member.
FIG. 38C is an illustration of a bone anchor variation of the device of FIGS. 38A and 38B.
FIG. 38D is a detail perspective view of the backing having an adjustable latch for attachment to the connecting member.
FIGS. 39A-39C show several views of another adjustable length variation in which an additional backing is used for affixing the device to soft tissue.
FIG. 40 is a perspective view illustrating a variation in which the backing is integrally formed with or attached to an elongated leash member.
FIGS. 41A and 41B are perspective views illustrating the delivery device for the tissue approximation device of FIG. 40.
FIGS. 42A to 42G illustrate the implementation of the tissue approximation device of FIG. 40, using the delivery device of FIGS. 41A/41B, to approximate and secure mid-face tissue.
FIGS. 43A to 43B illustrate the implementation of the tissue approximation device of FIG. 40, without using the delivery device of FIGS. 41A/41B, to approximate mid-face tissue.
FIG. 44 is a perspective view of the tissue approximation device of FIG. 40 with a low profile medical screw.
FIGS. 45A and 45B are side views of the low profile screw of the present invention.
FIGS. 46A and 46B illustrate the clipping off of the head portion of the low profile screw of the present invention.
FIG. 47 illustrates the implementation of the tissue approximation device of FIG. 40 underneath the eye using the low profile screw of the present invention.
FIG. 48 is a perspective view illustrating a variation in which the backing and leash member are integrally formed as a unitary elongated band.
FIG. 49A is a perspective view illustrating tines extending from both sides of the backing portion of the unitary elongated band.
FIG. 49B is a perspective view illustrating tines extending from both sides of the backing portion of the unitary elongated band, with tines on one side angled differently from those on the other side.
FIG. 49C is a perspective view illustrating tines extending from the leash member portion of the unitary elongated band.
FIG. 49D is a perspective view illustrating tines extending from both the backing and leash portions of the unitary elongated band.
FIG. 49E is a perspective view illustrating tines extending from side edges of the unitary elongated band.

### DETAILED DESCRIPTION OF THE PREFERRED EMBODIMENTS

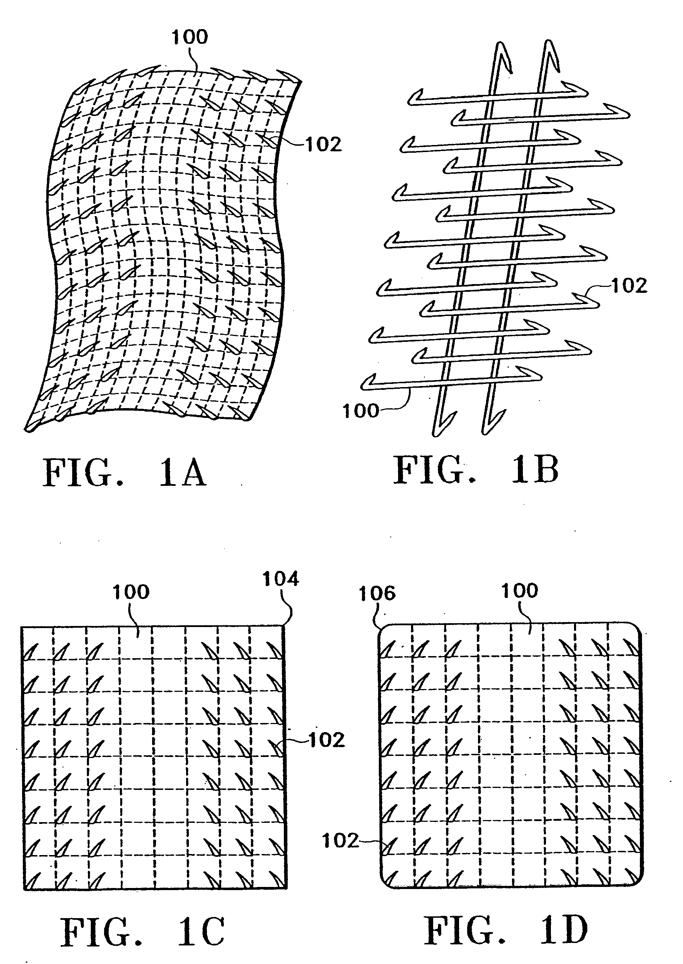

Our inventive device may be used when working with bone anchors or a variety of soft tissues. An exemplary device is shown in FIGS. 1A-1B and comprises a plurality of attachment points (102) emanating from and preferably affixed to a supportive backing (100) that is a generally porous material that may have the structure of a mesh, net, or lattice. The degree of flexibility of the backing is determined by the material of construction, the shape and dimensions of the device, the type and properties of the approximated tissue, and the area of the body into which the device is placed. For example, a tightly curved or mobile part of the body, e.g., a joint, will require a more flexible backing, as would a tendon or nerve repair due to the amount of bending the device needs for the attachment. Also, depending on the type of material used, the thickness of the backing as well as its width and length may determine the flexibility of the device. Furthermore, the backing may be prefabricated into different shapes as shown by the sharp corners (104) and rounded corners (106) in FIGS. 1C and 1D. The fabricated cross-sectional shape and dimensions of the mesh elements may vary to promote flexibility in regions of the backing. The cross-sectional shape of the mesh elements may be chosen to minimize local compressive stress between the backing and surface it rests upon, or have rounded and filleted edges to be less obtrusive to local circulation. The plurality of attachment points distribute tension over the contact area between the device and the tissue. The tension or forces are generally also distributed in the tissue and in the backing parallel to the interfaces between the tissue and the device.

Materials such as biodegradable polymers are preferably used to construct the backing and attachment points. Polymers synthesized from monomers comprising esters, anhydrides, orthoesters, and amides are particularly suitable for biodegradation. Examples of biodegradable polymers are polyglycolide, polylactide, poly-α-caprolactone, polydiaxanone, polyglyconate, polylactide-co-glycolide, and block and random copolymers of these polymers. Copolymers of glycolic, lactic, and other α-hydroxy acids are highly desirable. Although we prefer to use a single polymer or copolymer in a specific device, generally for ease of construction, the invention is not so limited. An example of an inventive device may be made of two or more types of polymers or copolymers (or molecular weights of the same polymer or copolymer). For instance, the backing material might be produced from a more flexible polymer and the points or tines of a stiffer material. The inflammatory response to these polymers is minimal, and they have been safely used in suture materials, stents, drug delivery devices, orthopedic fixation devices, and intestinal anastomotic rings.

Generally, we will refer to the soft tissue attachment points as "tines" or "prongs". These tines will refer both to points which are either sharp, i.e. able to separate tissue in a chosen use, or blunt, i.e. not able to separate tissue in that use. The attachment points may also be referred to as "barbs" when those points have the retaining point shown in several of the Figures discussed below. Generally, the tines, prongs or barbs penetrate into soft tissue and for a short distance. The attachment points preferably do not traumatize tissue in any major way, e.g., by penetration through a selected area of tissue to meet another device on the opposite side of the tissue. The attachment points may be considered to interlock with modulation in the adjacent soft tissue rather than penetrate as by a pin or bolt.

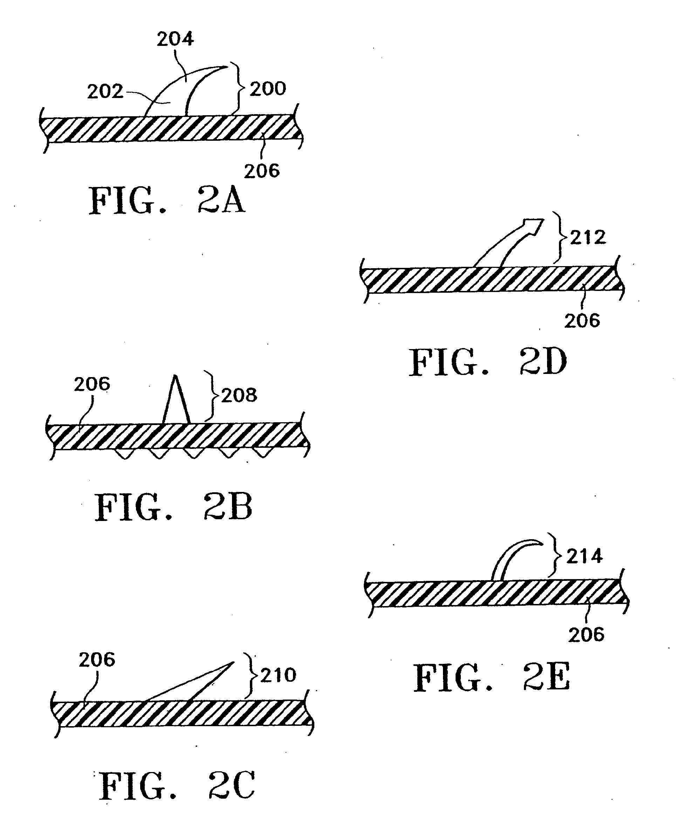

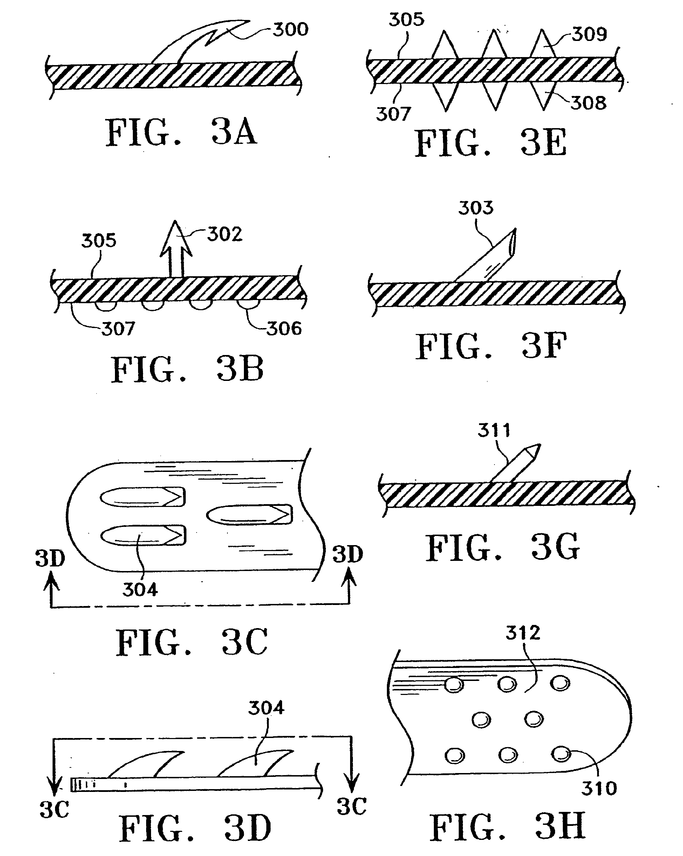

As shown in FIGS. 2A-2E, the shape of the attachment points or barbs may be varied depending, e.g., on the area of the body involved and the type of tissue requiring closure or reapproximation. The tines may be canted, erect, or curvilinear as necessary for a specific procedure. As shown in FIG. 2A, the tines (200) may have a wide base (202) that supports a projection (204) from the backing (206) against the degree of tension required to close a wound or approximate tissue. For example, the attachment points may be erect tines (FIG. 2B--208), canted tines (FIG. 2C--210), canted arrowheads (FIG. 2D-212), canted hooks (FIG. 2E--214), or may have a single straight cross-section (FIG. 3G--311) that is nail-like, that does not vary over the length of the prong, for example, similar in shape to a nail or sharpened pencil. Furthermore, the tip of the attachment points may be varied as shown in FIGS. 3A-3D. The tips may be barbed (300 in FIG. 3A), arrowhead (double-barb) (302 in FIG. 3B), or cheese grater (304 in FIG. 3D). A side view of the cheese grater tips is shown in FIG. 3D. A faceted tip (303 in FIG. 3F) is shown. The faceted tip is especially desirable where the force to penetrate tissue is normal to the tissue surface.

The connection of the prong to the backing may be rounded or filleted, or the backing built-up around the prong, to reduce structural stress concentrations. The backing or connecting structure may branch out away from the center, with each branch in turn branching to grapple tissue in a distributed fashion. All edges of the device may be smooth except where sharpness is needed at the tip of the prong to pierce into the tissue. Once the prongs pierce into the tissue, the tissue may become supported against the backing to minimize additional piercing or irritation by the prong tip. The device may be molded, stamped, machined, woven, bent, welded or otherwise fabricated to create the desired features and functional properties.

The device may also have attachment points both on its front side (305) and on a back side (307). As shown in FIGS. 3B and 3E, the front and back sides have attachment points. The attachment points on the front side (309) generally approximate tissue. The attachment points on the back side (307) are auxiliary attachment points that may comprise forms such as round nubs (306) or pointed nubs (308). The auxiliary attachment points may be used to secure or promote stable implantation of the device. Soft tissue may be gently pressed into open regions of the backing thereby helping to fix the device in place against both underlying and overlying tissue after the modulation or interlocking of skin. FIG. 3H shows a reverse view of the nubs (310) on the back side of the device (312). The attachment points on a two-sided device are not limited to the combinations disclosed above, but may comprise any combination of the previously mentioned attachment point shapes and orientations.

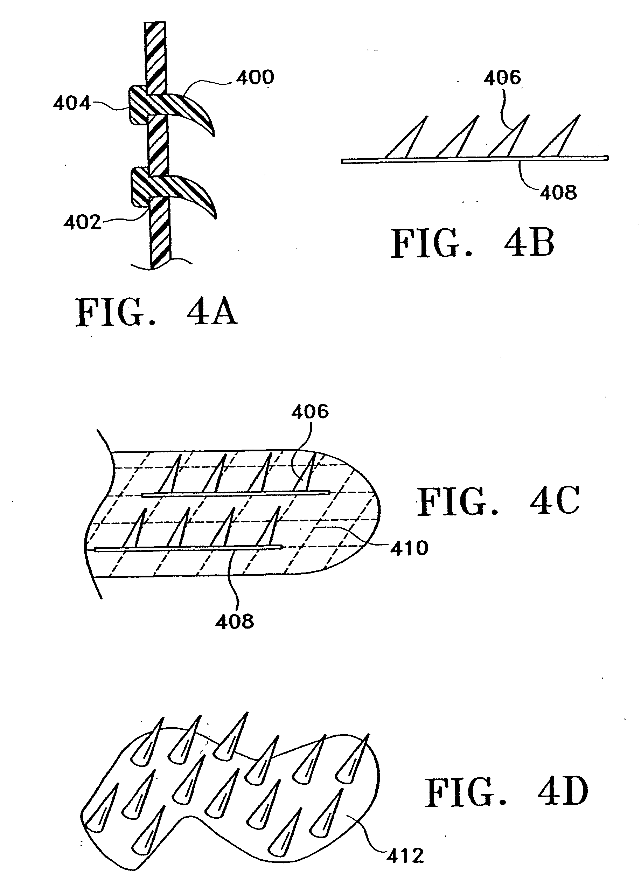

Structural variations can also be made to the backing of the device. As shown in FIG. 4A, the attachment points (400) may be placed through a plurality of openings in the backing (402) and secured to the backing by a flange (404) or hub. In FIGS. 4B and 4C, the points (406) may also connect to strips (408) of the same material as the attachment points which are then secured to a backing (410). The backing may also be comprised of a solid material (412) instead of a porous material.

The extent of porosity, or total surface area may be used to control the absorption rate of the device, and may also be used to optimize the strength-to-mass properties of the device, increasing the section modulus of structural cross-sections per unit mass. The backing structure may comprise partial folds, waves or grooves to help hold tissue against both surfaces of the backing. Regions of the backing may function as suction cups to help hold tissue to the backing.

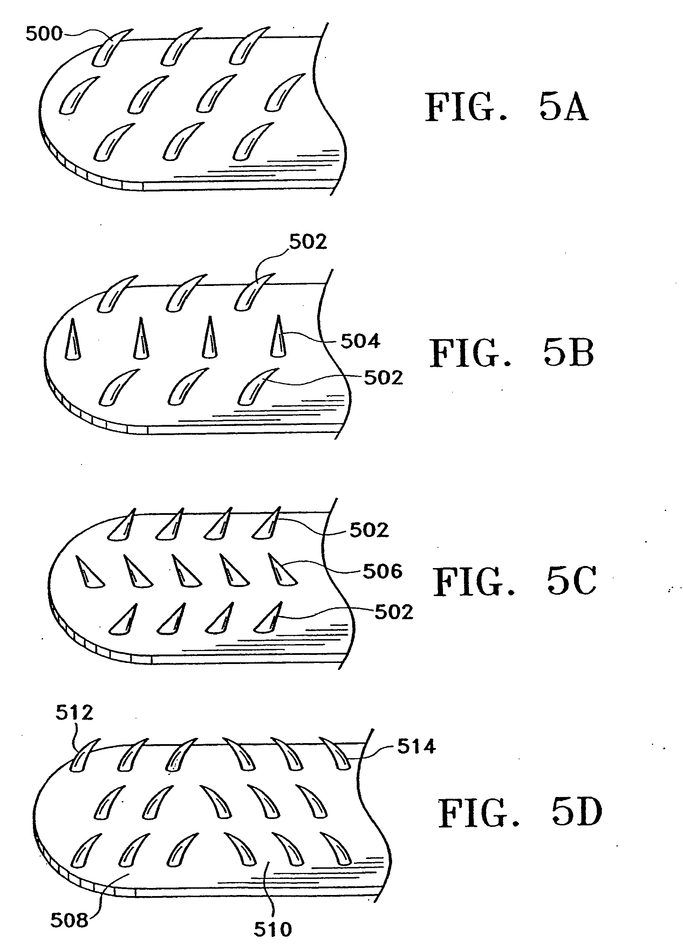

The density, distribution, length, and orientation of attachment points on the backing may be modified depending on the type of wound closure or tissue approximation procedure. Attachment points may be bent or curve gradually, with the tip directed at an optimal angle relative to the backing to aid device penetration and stability within the tissue, and to reduce tissue irritation after device installation. Attachment points may be canted in one direction (500), such as toward the center of the device as shown in FIG. 5A. The attachment points may also be variously oriented, such as toward center (502) and erect (504), or toward center (502) and away from center (506). The attachment points may extend in any relative direction or orientation on the backing. Or, as shown in FIG. 5D, the backing is divided into a first area (508) and a second area (510). Attachment points in the first area (512) and second area (514) are canted toward each other. The inventive device may also be sectioned into a plurality of areas, with each section being variously oriented to another section.

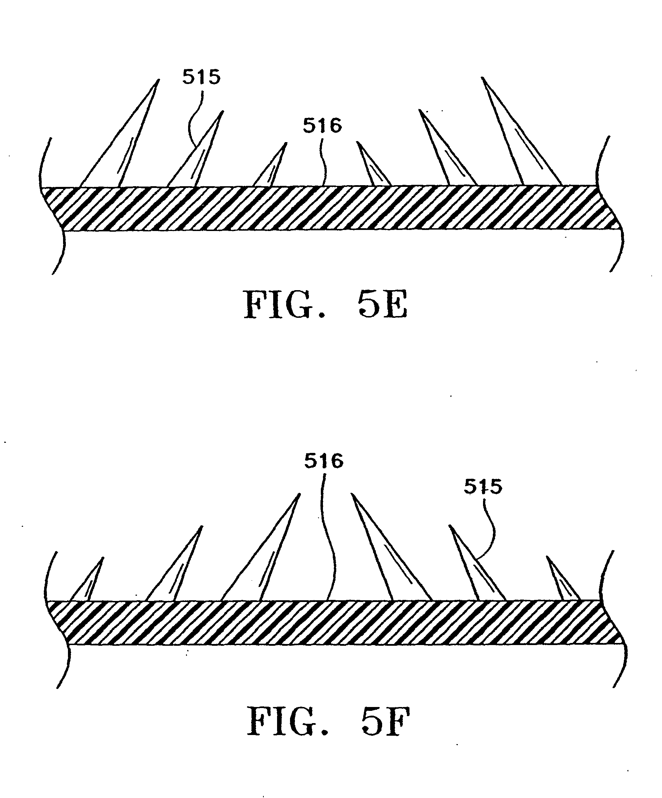

In another exemplary variation, attachment points of various lengths emanate from a single backing. For example, in FIG. 5E, the attachment points (515) are progressively shorter the closer they are to the center of the device (516). The attachment points (515) may also become progressively shorter the farther they are from the center of the device as shown in FIG. 5F. The variations shown in FIGS. 5B and 5C have regions of attachment points canted toward the center (502) and with other regions of attachment points with erect points (504 in FIG. 5B) or canted away from the other end (506 in FIG. 5C) of the device. These variations are more difficult to dislodge when situated in an area of the body having both to-and-fro movement, e.g., the inside of an elbow or back of the knee, or during placement of the device.

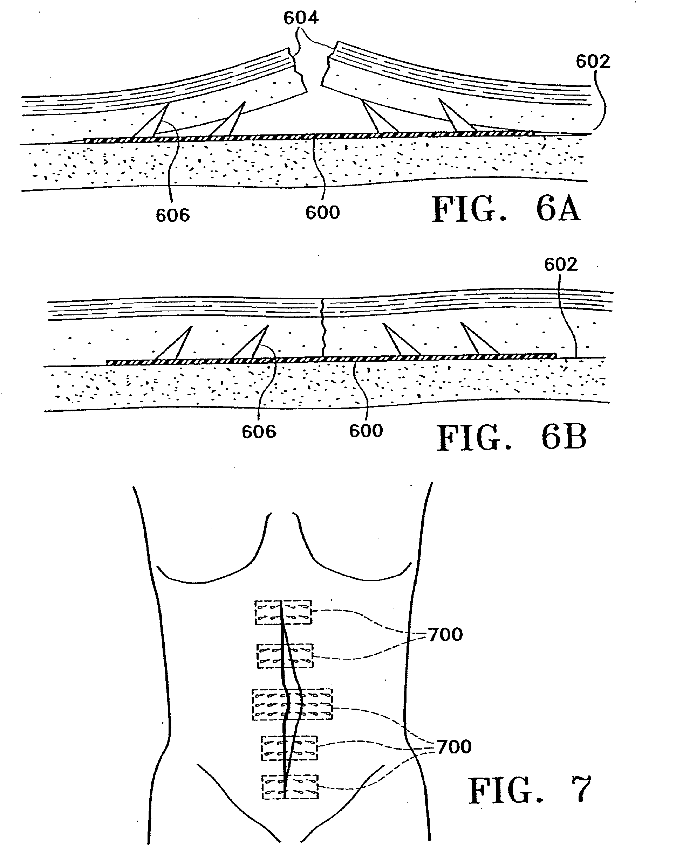

Portions of simple wound closures are shown in FIGS. 6A-6B. These wound closures involve placing the device (600) at the bottom of the wound, usually at the level of the sub-dermis (602). The edges of the wound (604) are approximated and then secured by fixation, e.g., by pressing, to the multiple attachment points (606). An example of the device placement in a laparotomy closure is shown in FIG. 7. The increased length of this incision requires placement of multiple devices (700).

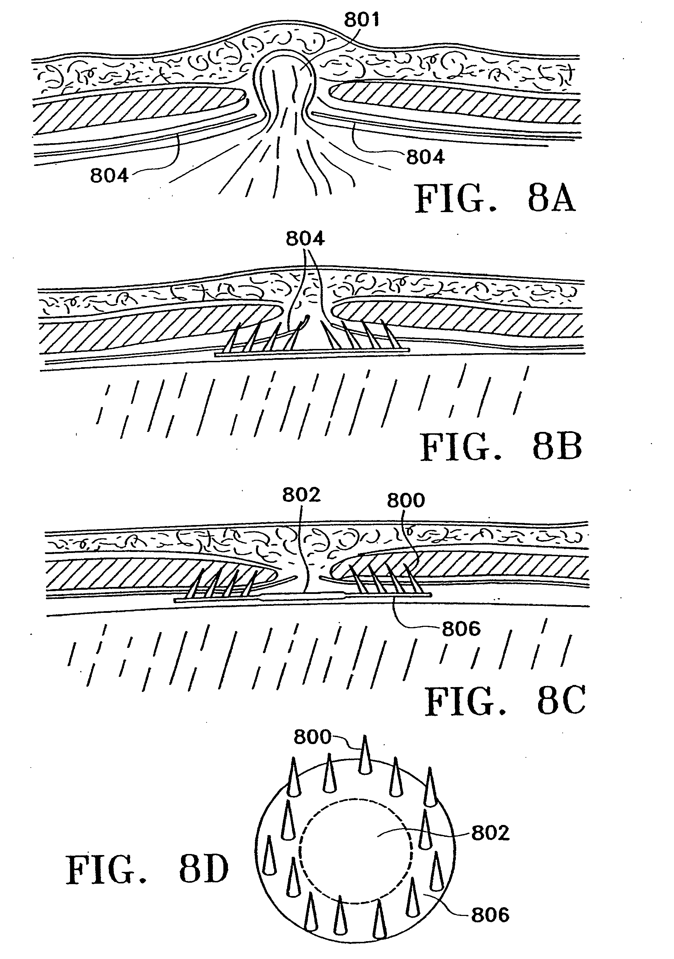

A unique application of this device occurs in hernia repair in which case the biomaterials are not absorbable but rather are more likely to be PTFE and POPU ("Gore-Tex"), polypropylene, or other permanent implant material. Once the hernia (801) is reduced, a device may be used to close the hernia defect by joining the edges of the separated fascia (804) as seen in FIGS. 8A and 8B. However, the device may also be modified to aid repair of a difficult hernia resulting from such circumstances as operating on an obese patient or large hernia, or having a wide fascial debridement where the fascial edges cannot be brought together. FIGS. 8C and 8D are variations of the inventive device that may be used in these cases. The attachment points (800) are secured to the ends of the backing (806) and are still used to adhere the device to tissue, but the points are spaced so that the central area of the backing is a flat surface without points (802) that covers the defect. The device in FIG. 8D is preferably used in an incisional hernia repair.

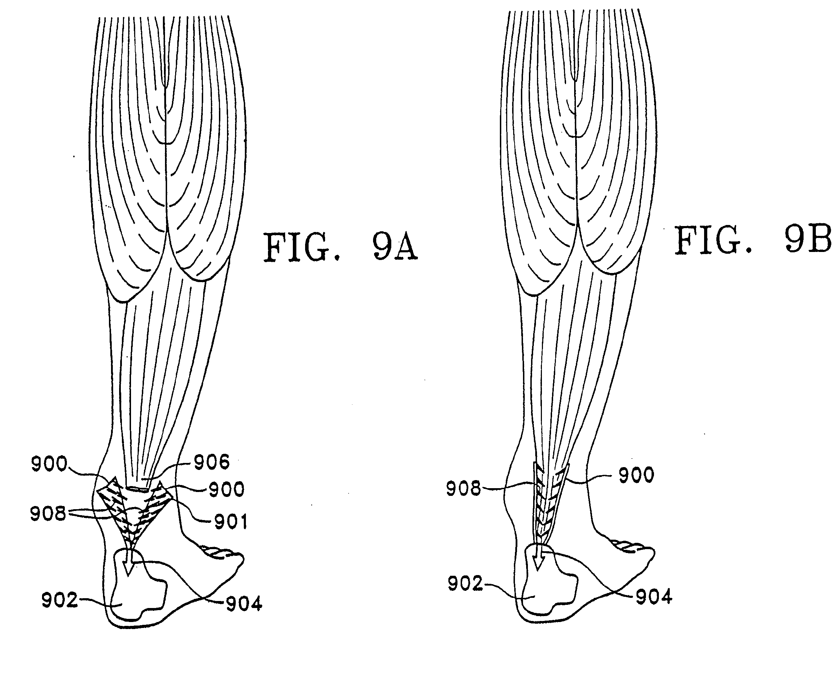

The device may also be constructed to reattach soft tissue such as tendons and ligaments to bone, as well as other soft tissue such as cartilage and the free ends of vessels or nerves. In FIG. 9A, the inventive device functions similar to a clamp. Backings with attachment points (900) are sides of a clamp that has a first end (901) and a second end (904). The first end (901) grasps tissue and the second end (904) is an anchor for tissue. For example, a ruptured tendon (906) may be fixed to the attachment points (908) of the first end of the clamp (901) and approximated to bone (902) with an anchor such as a pin or nail at the second end of the clamp (904), as seen in FIG. 9B. After mechanical fixation of the tissues, the biochemical phase of the wound healing process will begin, eventually forming a natural union between tendon and bone. Ligament and cartilage to bone unions using the device would undergo the same mechanical and biochemical processes.

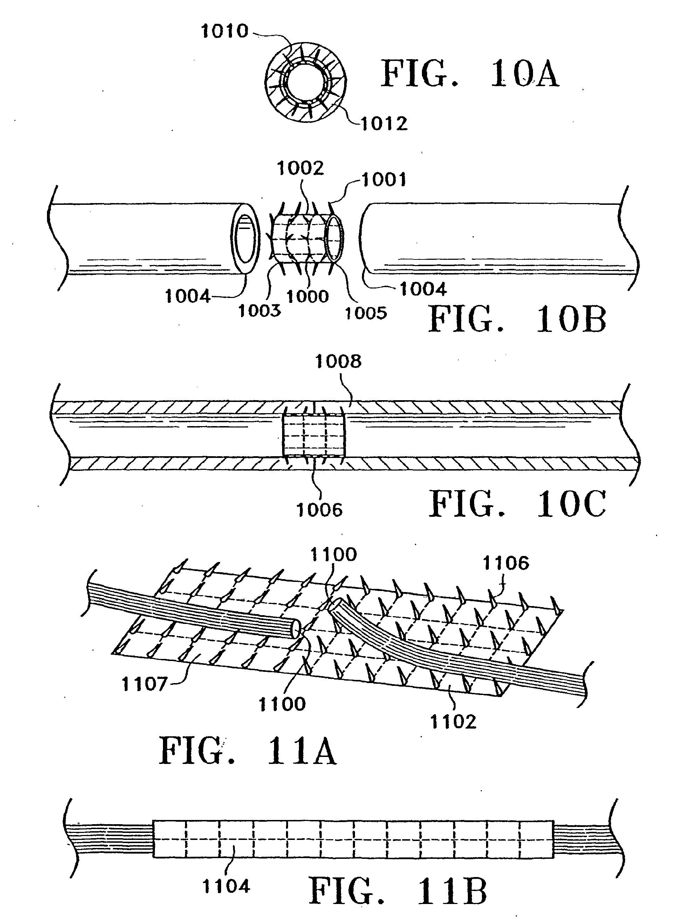

Vascular anastomoses may also be constructed with the device. In FIG. 10B, the backing has a tubular shape (1000) with attachment points (1001) on the outside surface (1002). The outside surface (1002) has a first end (1003) and a second end (1005) that opposes the first end (1003). The free ends of a vessel(s) (1004) are placed over the device, creating an anastomosis (1006) that is secured by attachment points fixed into the wall of the vessels (1008). The attachment points are preferably pointing towards the anastomosis (1006), with the attachment points on the first end (1003) being canted toward the second end (1005) and vice-versa. An axial view of the relationship of the attachment points (1010) to the vessel wall (1012) is shown in FIG. 10A.

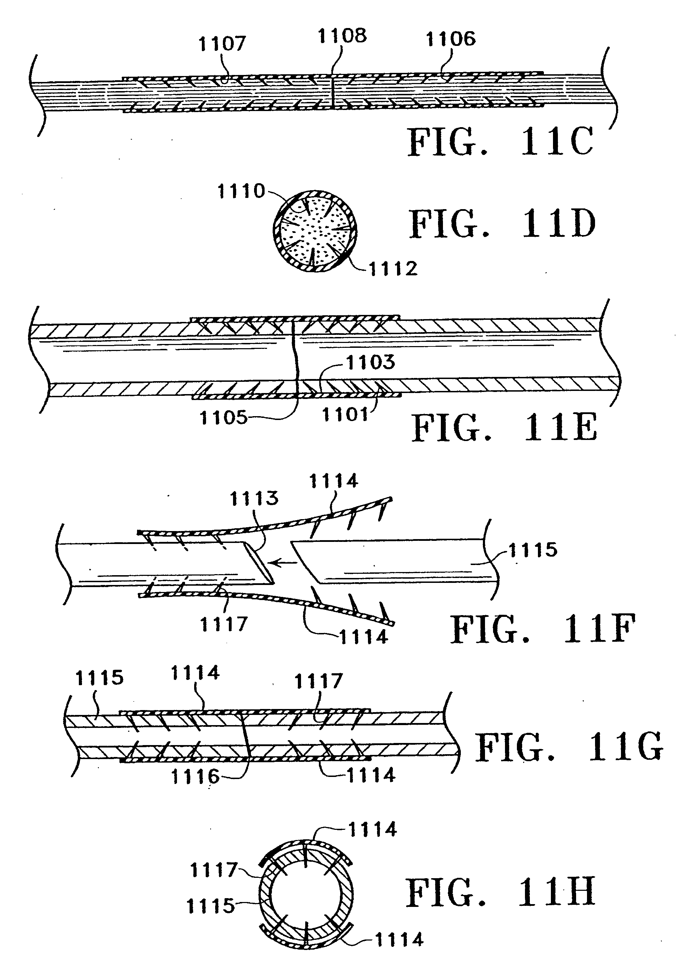

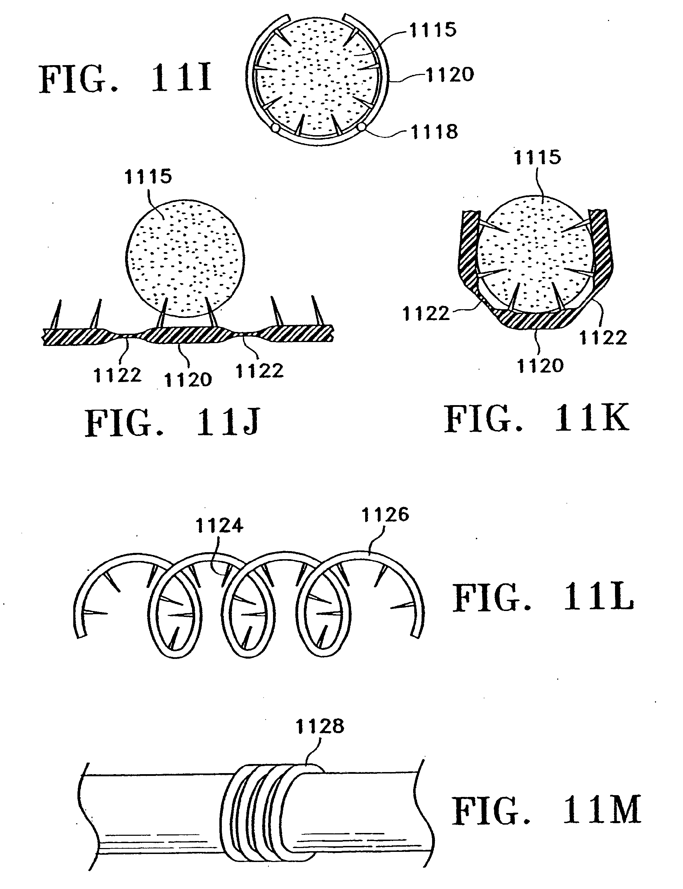

Vessels and other soft tissue such as nerves, cartilage, tendons, and ligaments may also be joined as seen in FIGS. 11A and 11B. Two ends of tissue (1100) are brought and held together by the backing and attachment point construct (1102) being wrapped around the circumference of the tissue (1104). The attachment points (1106) are on the inside surface of the backing (1107) and secure the union at a central region (1108) as seen in FIG. 11C. An axial, cross-sectional view of the relationship between the attachment points (1110) and tissue (1112) is shown in FIG. 11D. The resulting form is, i.e., a tubular structure that has an inside surface (1107) with a central region (1108). The attachment points on the inside surface (1106) may be canted toward the central region (1108). FIG. 11E shows the device with attachment points (1101) on the inside surface of the backing (1103) being wrapped around vessel ends to create an anastomosis (1105). Instead of being wrapped around tissue, edges (1113) of tubular structures (1115) can also be joined by externally placing 2 or more strips of backing of a device (1114) on approximated tissue as shown in the side views of FIGS. 11F-11G, and the axial view in FIG. 11H. The attachment points (1117) also point toward the area of tissue approximation (1116).

FIGS. 11I-11M are additional exemplary variations which vary the mechanisms used to improve device flexibility. In FIGS. 11I-11K, the backing has areas of comparatively higher flexibility than other areas of the backing. In an axial view of the variation in FIG. 11I, the backing is equipped with hinges (1118) that allow bending of the backing (1120) around tubular soft tissue structures (1115). In a second variation, the amount of material in the areas of the device that fold (1122) is reduced as shown in FIGS. 11J-11K. Another variation is seen in FIGS. 11L-11M where attachment points (1124) of a device extend from a backing in the form of a coil or spring (1126). The edges of soft tissue are approximated when the coil or spring is reduced (1128).

### Device for Brow and Face Lift Procedures

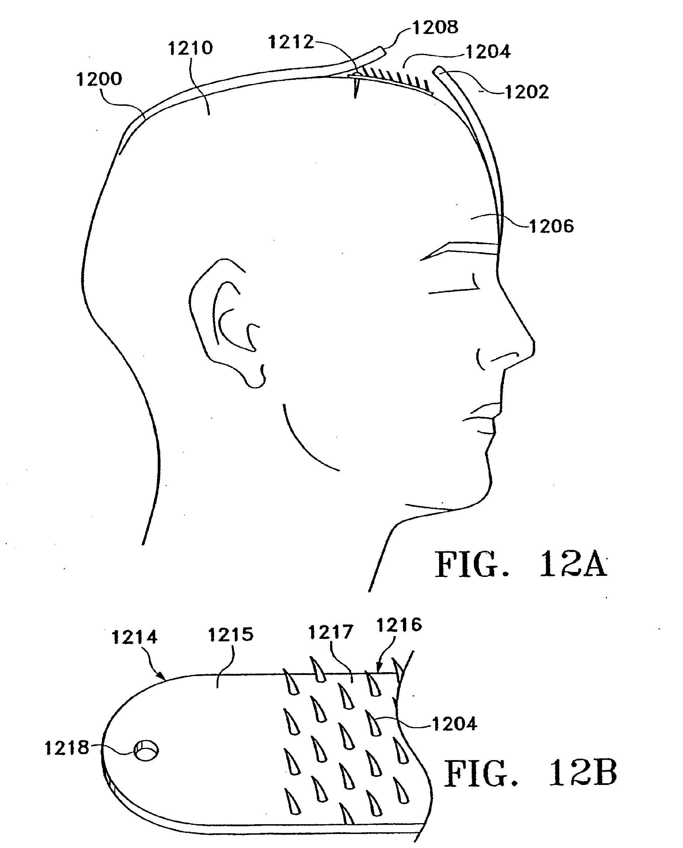

The device may also be used in soft-tissue remodeling, such as a brow-lift, shown in FIG. 12A. After dissection of the scalp (1200), the anterior scalp flap (1202) may be raised over the attachment points (1204) to lift the brow (1206). The ends of both the anterior flap (1202) and posterior flap (1208) may then be trimmed and fixed onto the attachment points (1204) to close the wound. The device may be secured to the skull (1210) by a screw (1212). The inventive device in this example may have a first end (1214) and a second end (1216), the first end having a first area (1215) and the second end having a second area (1217). The first area (1215) and second area (1217) may have extending attachment points (1204) or one or more openings (1218) to accommodate a screw(s) (1212). The second area attachment points are canted toward the first end of the device as shown in FIG. 12B.

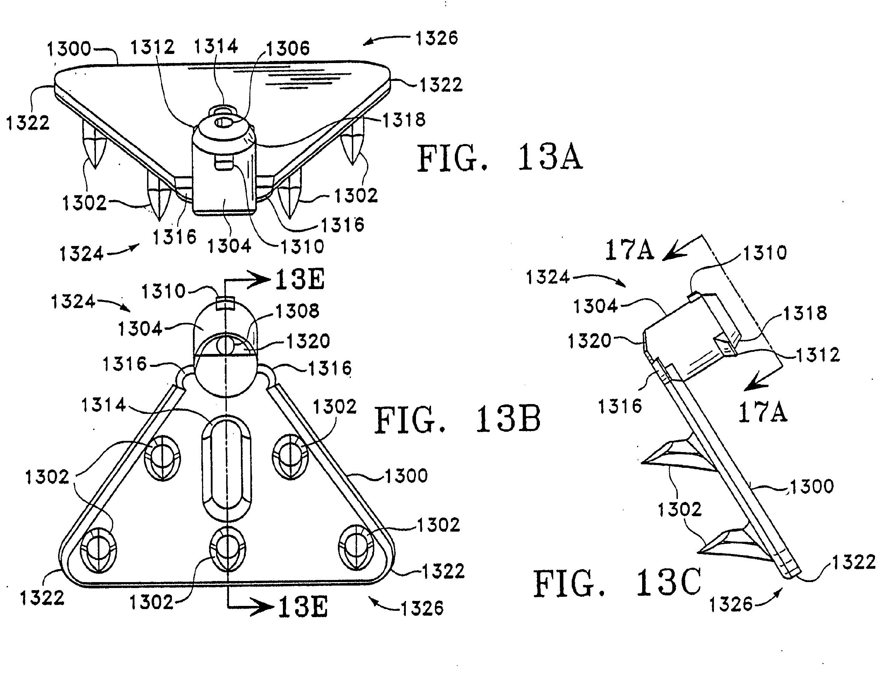

FIGS. 13A-13C show an alternative variation of the device which may be used in a brow-lift or similar surgical procedure. This device may generally be inserted under a patient's scalp while securely interlocking a small portion of the scalp to the device preferably via a plurality of attachment points. It may also be designed generally to lay against the cranium in a low profile while secured to the cranium to provide a brow lift. This variation comprises supportive backing (1300), which is shown substantially as an equilateral triangle, or in a delta shape. Backing (1300) may be any of a wide variety of triangular shapes, e.g., isosceles, etc. which functions to distribute planar loads equally radiating from a small area, e.g., post (1304). Various alternative shapes are discussed below in greater detail. Post (1304) is functionally for the maintenance of the device in place; other sections of the surgical procedure used to support the device in a specific part in the body. Post (1304) is placed on the side of the body opposite to the tines.

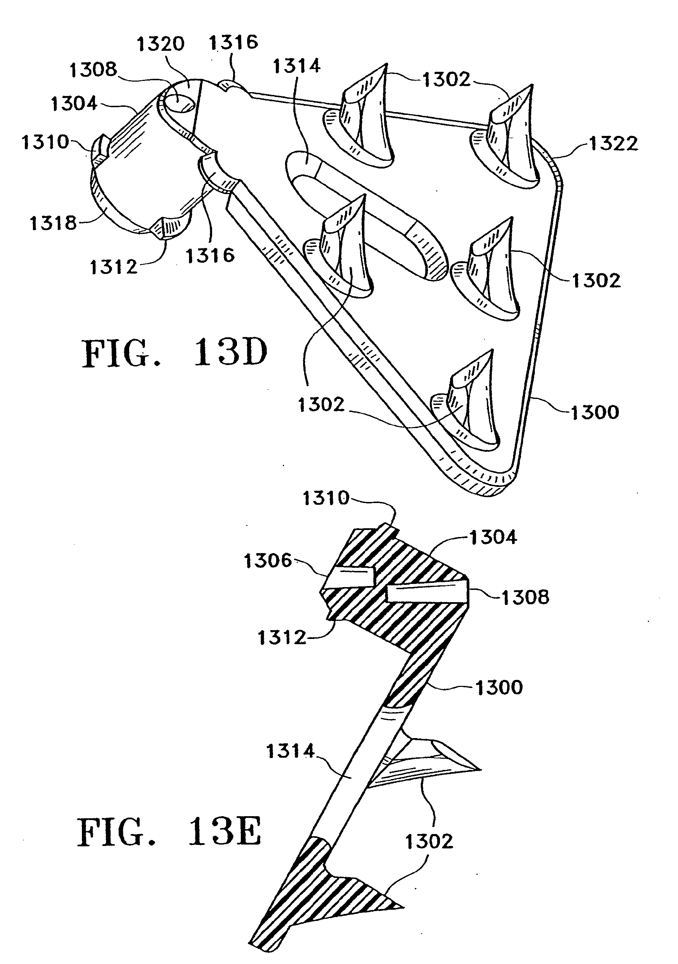

FIG. 13A shows a front side view of supportive backing (1300) that may be used for either fixation or anchoring. This variation may incorporate sharp corners at the triangle vertices, but preferably has radiused or rounded corners (1322) to aid in reducing abrasion and cutting in adjacent tissue. An anchoring post (1304) may be located at one of the vertices of backing (1300). This anchoring post (1304) is shown in this variation as being substantially perpendicular to a plane of backing (1300), but may be other shapes as discussed below. Moreover, this device may be made of any of the materials discussed herein, and is preferably comprised of a biodegradable or bioabsorbable material but is obviously not limited by material type. For instance, the device may be comprised of certain biological materials as well, e.g., collagen, hydroxyapatite from both natural and synthetic sources, bone graft, or any combination or polymerized version of these materials. FIG. 13D shows more clearly a perspective view of a preferred variation of the device shown in FIGS. 13A-13C.

In this variation, supportive backing (1300) may comprise a triangular form having a first end (1324) and a second end (1326). This variation may typically be comprised of a front side, as shown in FIG. 13A, and a back side, as shown in FIG. 13B. On the front side, preferably near a vertex of the triangular shape, is an anchoring region. This region may comprise anchoring post (1304) as seen in FIGS. 13A-13C, and this anchoring post (1304) may be a variety of shapes, e.g., a hook or an angled post, etc., but is preferably a perpendicular post having a proximal and a distal end. Moreover, post (1304) is preferably integral with backing (1300) so as to be formed from a single piece. This allows the device to be formed entirely into a single integral device by various manufacturing methods, e.g., injection or die molding. Post (1304) may also be a separate structure fixedly attached to backing (1300) by any variety of fastening methods, e.g., mechanical fasteners or adhesives. The distal end of post (1304) may be chamfered (1318), as shown in FIGS. 13A and 13C; this would provide a degree of tolerance to enable the surgeon to easily locate and insert post (1304) into a receiving hole without sacrificing device integrity.

Post (1304) may preferably further comprise a locking device proximal of chamfer (1318). This locking device may utilize a variety of locking mechanisms but is shown in this variation as front tab (1310) and partial collar (or rear tab) (1312). The locking mechanism is preferably integral with post (1304) and may have a diameter which is greater than a diameter of post (1304). In any case, partial collar (1312) is preferably elastically deformable, but may also be plastically deformable. Such deformability allows front tab (1310) and partial collar (1312) to compress upon insertion into a patient's skull and subsequently be able to spring back upon full insertion to provide a friction-fitted locking or securing feature. The locking device may alternatively be a locking key mechanism or any conventional locking mechanism. However, the locking mechanism may be omitted entirely because the device bases much of its stability, once inserted into a patient's cranium, upon the downward forces applied by the overlying tissue. Thus, much of the forces acting on the device apply bending loads on post (1304) rather than axially-oriented tensile loads.

As seen in FIG. 13A, post (1304) may incorporate a distal channel or cavity (1306) which may extend partially into the post from the distal end or entirely through the post. This distal cavity (1306) may have a diameter which is smaller than the diameter of post (1304) and may be aligned along an axis defined by post (1304) or may extend at an angle within post (1304). The cross-section 13E-13E of FIG. 13B is shown in FIG. 13E and shows more clearly the orientation of distal cavity (1306) within post (1304) for this variation. Distal cavity (1306) may aid in reducing the amount of material used in the manufacture of the device, and is particularly useful in imparting a desirable degree of flexibility to post (1304) which may facilitate the insertion of post (1304) into the cranium.

Post (1304) may further define another hole, proximal cavity (1308), which may be used for tooling purposes as well as further adding to the flexibility of post (1304). Proximal cavity may extend from chamfered proximal end (1320), which may also aid in tooling and helping to prevent tissue abrasion. Proximal cavity (1308) may be non-concentrically located relevant to distal cavity (1306) and as shown in FIG. 13E, may extend partially into post (1304) or may be a through-hole extending entirely through to the distal end of post (1304). Although proximal cavity (1308) may not necessarily be required, it may be utilized in a variety of ways. For example, proximal cavity (1308) may be used for aligning the device for tooling during manufacture, or it may also be used as a location to allow a user or surgeon to manipulate the device using tools for placement of the device within a patient. This proximal cavity (1308) may have a diameter, e.g., about 1 mm, which is smaller than a diameter of post (1304).

In addition to proximal cavity (1308), the device may also comprise protrusions, tabs, or "ears" (1316), as seen in FIGS. 13A-13D. These protrusions (1316) are preferably integral with backing (1300) and may generally be located anywhere on backing (1300), but is preferably located near first end (1324). FIG. 13B shows protrusions (1316) located on either side of post (1304) and may provide a surface for manipulating the device by the doctor or surgeon either during placement into the patient or during removal.

FIGS. 13A and 13C show the front and side views, respectively, of attachment points (1302). As discussed above, attachments points (1302), also called "tines" or "prongs" are preferably integrally affixed to backing (1300) but may also be separately attachable. They are preferably located on the back side of backing (1300), i.e., the side opposite of post (1304), and are preferably angled towards first end (1324). Moreover, individual attachment points (1302) may be of varying sizes and angles depending upon the desired securing effect. Attachment points (1302) are discussed in greater detail above. In this variation, individual attachment points (1302) may vary in density, but are optimally spaced relative to one another. Factors for optimizing attachment point relative placement may comprise the ease of securing tissue to attachment points (1302) and the distribution of loads generated by the attached tissue over each of attachment points (1302). For instance, if attachment points (1302) were located too closely to one another, piercing the tissue would be difficult because of the distribution of stresses on the tissue to be pierced by attachment points (1302).

Another example may include having an increasing number of attachment points (1302) placed on backing (1300) the farther they are located from front end (1324), where the greatest number of attachment points are located in the direction of tensile loads on the device. The spacing between individual points (1302) may be functional in that the number, density, and placement of points (1302) are optimized to evenly distribute the loads, e.g., shearing forces and bending moments, generated by the attached scalp in a brow-lift procedure. Moreover, attachment points (1302) are preferably configured to penetrate partially through the soft tissue. For instance, the sharpness of attachment points (1302) are such that they allow easy penetration through the periosteum.

FIGS. 13B and 13D show supportive backing (1300) which may also comprise through-hole (1314) that is defined within backing (1300). Through-hole (1314) may generally be any shaped hole but is shown in this variation as being slotted. Through-hole (1314) serves several functions which may include reducing the amount of material used in manufacturing the device, it may also add desirably to the flexibility of backing (1300). Additionally, through-hole (1314) maybe configured as an alignment aid for tooling purposes. In addition to aligning, through-hole (1314) may also serve as a surface for a tool to grasp during device placement or removal. Flexibility is preferable because it enables backing (1300) to bend and conform more closely to the shape of the patient's cranium against which the device is placed. The degree of flexibility of backing (1300) may be tuned to a predetermined degree depending upon several factors, e.g., the configuration and size of through-hole (1314). Although shown as a slot, backing (1300) may define virtually any through-hole shape which serves the functions discussed above, i.e., increasing backing (1300) flexibility and aiding in tool alignment.

### Exemplary Method of Installing and Securing

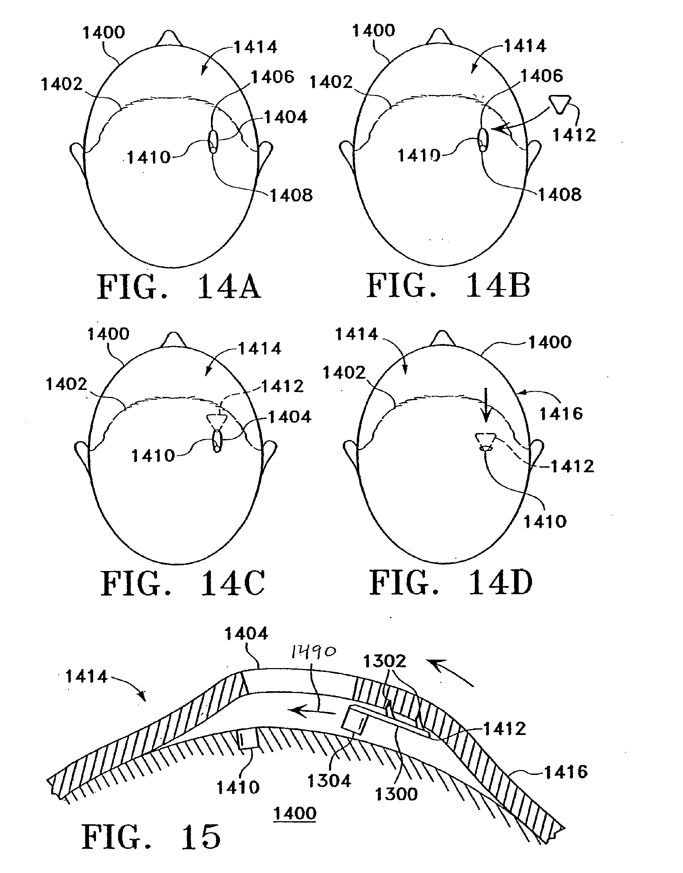

FIGS. 14A-14D illustrate an exemplary method of installing the device of FIG. 13A. The top of a patient's head is shown having a hairline (1402). As seen in FIG. 14A, the doctor or surgeon may initially make an incision (1404) in scalp (1414) preferably along a sagittal plane defined by cranium (1400). The incision (1404) may typically be done in the patient's hairline, if possible, to minimize any visible scarring which may result. The length of incision (1404) is typically determined by the length or amount of scalp the patient may desire or the surgeon may determine necessary to be lifted for a successful brow-lift procedure. This incision length may generally range from about 1 to 2 cm but may be more or less depending on the desired results.

Once incision (1404) is made, a hole (1410) may be drilled within cranium (1400) at the incision second end (1408). Hole (1410) drilled into cranium (1400) may typically be about 4.0 mm in diameter and may be made by a conventional surgical drill (not shown). As shown in FIG. 14B, once the incision and hole are made, a device (1412) may be inserted between cranium (1400) and scalp (1414) at the incision first end (1406) such that post (1304) faces towards cranium (1400) and attachment points (1302) face the underside of scalp (1414), i.e., subperiosteal. FIG. 14C shows an outline of device (1412) placed at incision first end (1406) and beneath scalp (1414). Once device (1412) has been inserted, the portion of the scalp tissue to be raised (1416) is set on device (1412) via attachment points (1302). FIG. 15 shows a cross-sectional view of FIG. 14C where the tissue to be raised (1416) has been set on attachment points (1302). Once tissue (1416) is set, a force (1490) may be applied to device (1412) preferably via post (1304). Force (1490) then draws the device (1412) and tissue (1416) towards hole (1410) which is configured to receive post (1304). As shown in FIG. 14D, once post (1304) is secured within hole (1410), force (1490) may be removed, thereby leaving the brow desirably lifted. Alternately, the device (1412) can be secured to the cranium (1400) before the tissue (1416) is set on the device (1412).

Once device (1412) has been installed, attachment points (1302) and post (1304) undergo shear and bending loads from the lifted tissue (1416) pulling on the device (1412). However, these loads may decrease rapidly and approach zero as scalp (1414) heals. This decrease in loading may take up to about six weeks, but device (1412) may stay in place beneath scalp (1414) for several weeks up to several years, with sufficient strength for about six weeks, to prevent scalp (1414) from moving excessively during the healing process and thereafter being absorbed by the body, thereby removing the necessity for a second procedure to remove device (1412).

### Variations on Attachment Points

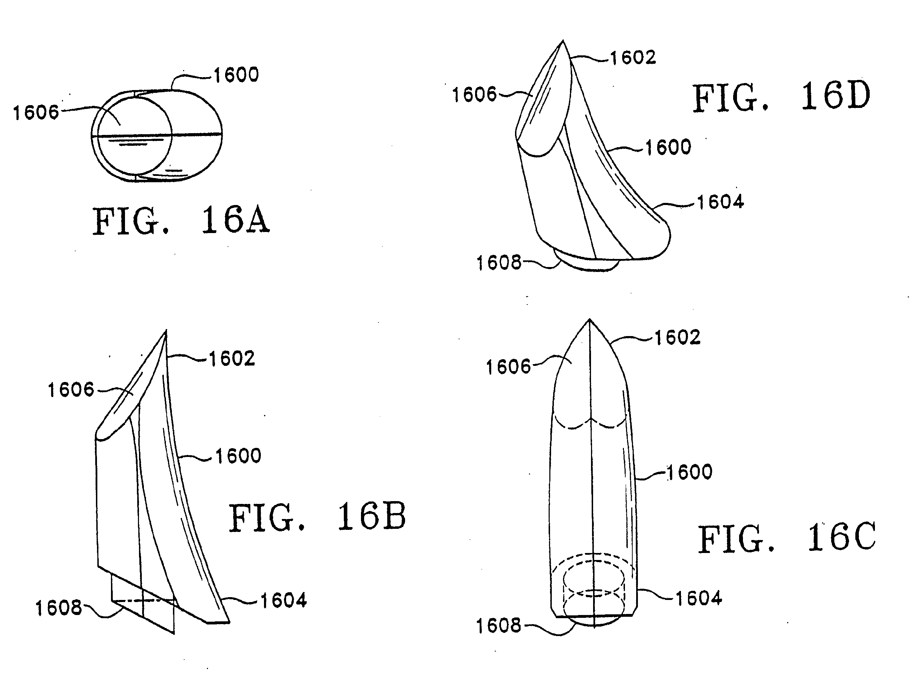

FIGS. 16A-16D show a preferred variation for attachment points. FIG. 16A shows a top view of a single attachment point (1600) having a swept face (1606). FIG. 16B is a side view of attachment point (1600) comprising distal pointed end (1602) and proximal base end (1604). Although any variations of attachment points discussed above may be used on the device, this variation is preferable because it is able to readily pierce tissue through the periosteum and simultaneously secure the tissue solidly by resisting any bending moments. In particular, swept face (1606) may be specifically faceted so that face (1606) is preferably oriented to be essentially perpendicular to the plane of the tissue or scalp being penetrated, even though the tine axis defined by attachment point (1600) may not be perpendicular to the plane of the tissue or scalp.

Attachment points of this variation may optionally be manufactured individually and separately from the supportive backing and then individually attached via backing attachment (1608) to the backing by a variety of fastening methods, e.g., friction fitting, adhesives, etc. Optional backing attachment (1608) is seen in FIG. 16B, and more clearly in the back view of FIG. 16C. FIG. 16D shows the variation more clearly in a perspective view. Attachment point (1600), as mentioned, may be manufactured separately and attached, but it is preferably made integral with the device. Integrating the attachment point(s) (1600) with the backing not only provides uniformity in material type but also eliminates contact interfaces, which in turn may provide superior material strength and resistance to bending.

As discussed above and as shown in FIGS. 13A-C, attachment points (1600) are preferably manufactured or attached so that they are all substantially canted in parallel towards the post. However, the attachment points are faceted such that the tips of attachment points (1600) are effectively perpendicular to the tissue to be penetrated. Attachment points (1600) may also be manufactured or assembled so that they point in different predetermined directions, depending on the desired application. Furthermore, attachment points (1600) may optionally be made of varying sizes, as discussed in further detail above.

### Variations on Anchors

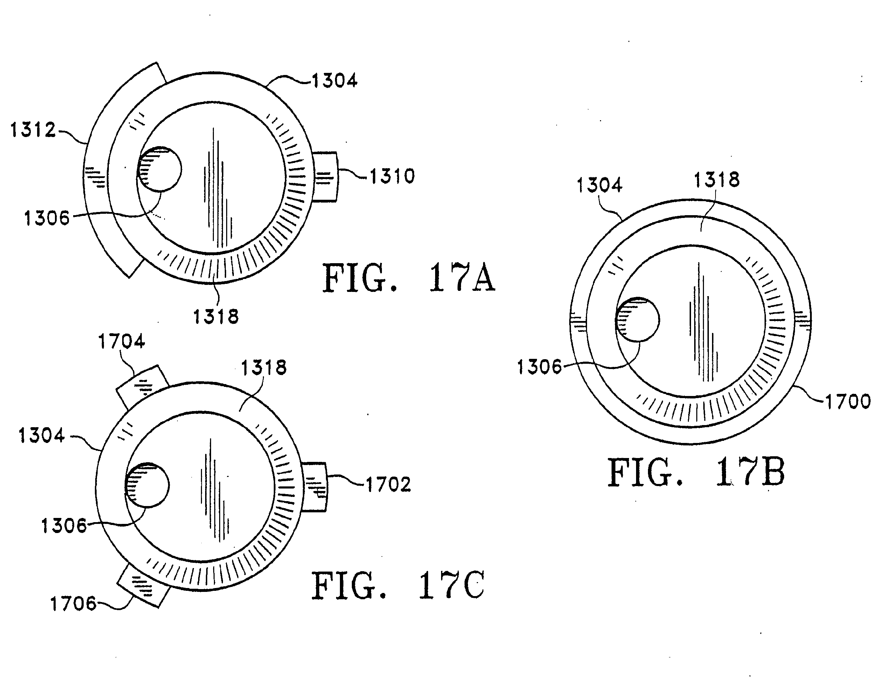

FIG. 17A shows perspective 17A-17A from FIG. 13C of the distal end of post (1304). As shown, partial collar (1312) and front tab (1310) preferably comprises integral extensions or protrusions which act as a locking device. Both partial collar (1312) and front tab (1310) may be plastically deformable but is preferably elastically deformable. The protrusions provide opposing forces upon insertion into the skull to produce a friction fit which secures the device in the patient. Partial collar (1312) may essentially circumscribe any predetermined percentage of the circumference of post (1304), provided that a sufficient fit is produced.

Aside from partial collar (1312), post (1304) may alternatively use locking mechanisms comprising barbs and sub-cortical wings. Moreover, post (1304) may also be threaded so as to be rotated, or screwed, into a threaded mating hole located within the patient's cranium.

FIG. 17B shows an alternative locking configuration from FIG. 17A. Here, partial collar (1312) is replaced by full collar (1700), which is preferably integral with post (1304) and may also be plastically or elastically deformable. A further variation for a locking configuration is shown in FIG. 17C, in which first, second, and third tabs (1702), (1704), (1706), respectively, replaces partial collar (1312). Again, tabs (1702), (1704), (1706) are preferably integral and elastically deformable, although they may also be plastically deformable. Essentially any locking configuration may be utilized by a doctor or surgeon depending upon the desired fit of post (1304).

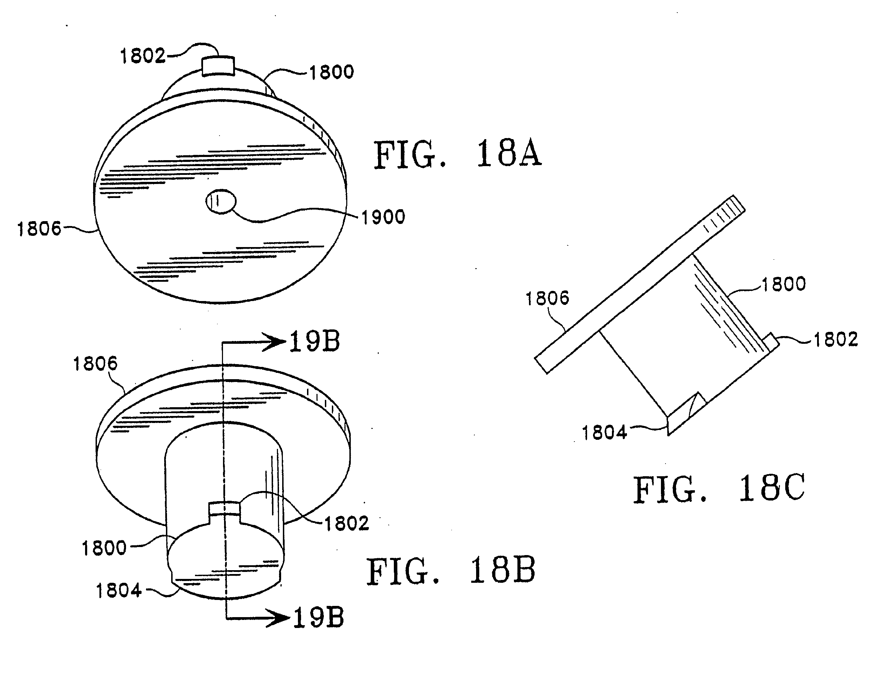

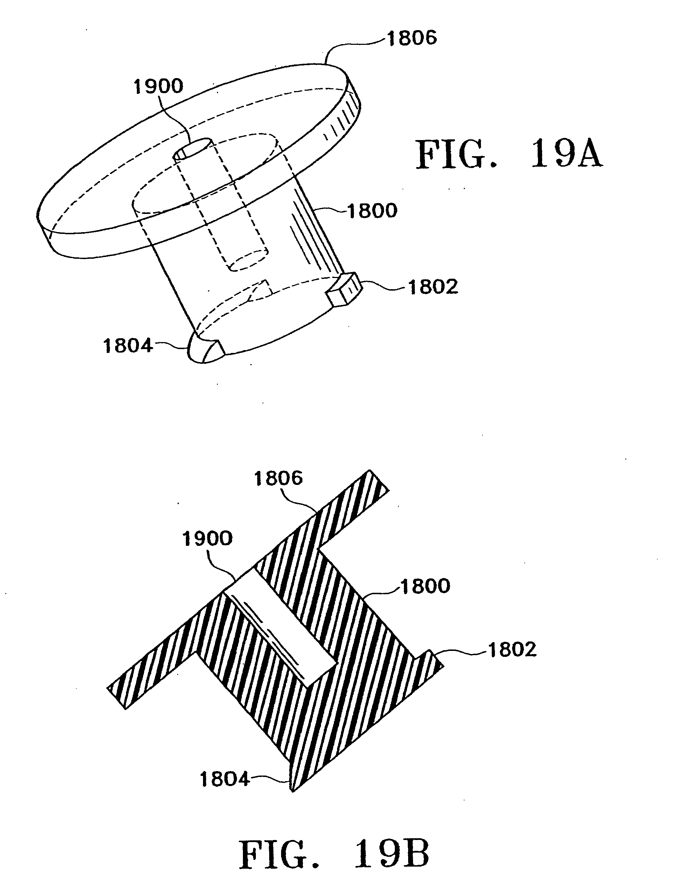

Aside from varying locking mechanisms, the flexibility of the post may be varied as well. As mentioned above, cavities may be disposed within the post to increase the post flexibility. FIG. 18A shows a back view of a variation of the cavity from FIG. 13B. As seen in FIGS. 18B and 18C, post (1800) is similar in most respects to the post shown in FIG. 13B. Post (1800) is illustrated extending from backing (1806), which is partially shown merely for clarity, with front tab (1802) and partial collar (1804). However, FIG. 18A shows a single axial cavity (1900) disposed within and extending from a proximal end of post (1800). FIG. 19A shows a perspective view of post (1800) from FIGS. 18A-18C where axial cavity (1900) is axially disposed within post (1800) and extends partially through. Cavity (1900) may extend through post (1800) perpendicularly to backing (1806) and concentrically along an axis defined by post (1800), but it may also extend off-axis and at an angle, as shown in FIG. 13E. Furthermore, cavity (1900) may also extend entirely through post (1800) as a through-hole. FIG. 19B shows the cross-section 19B-19B taken from FIG. 18B clearly showing cavity (1900) extending partially into post (1800).

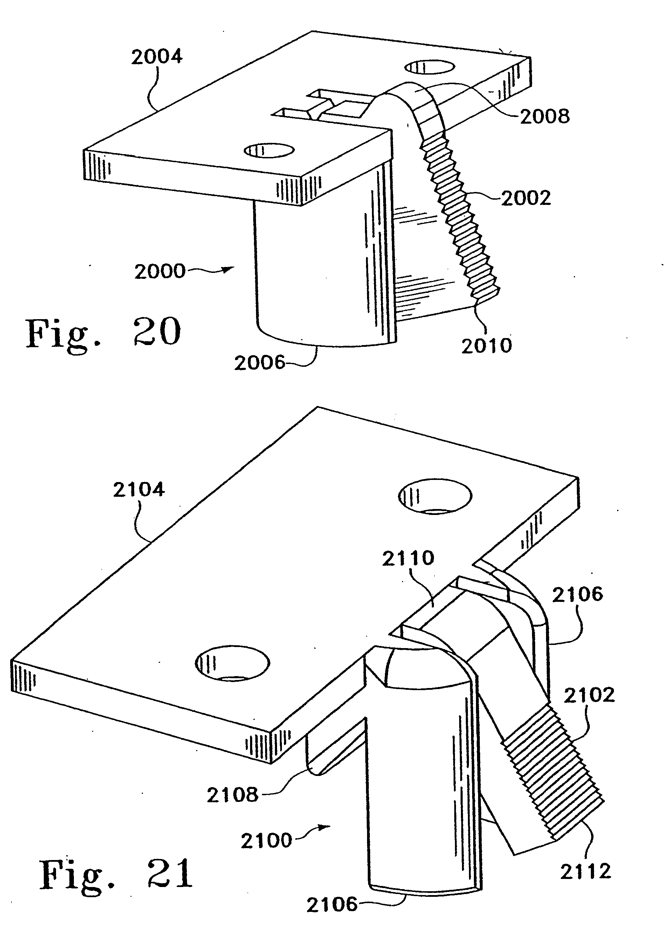

Another variation on the post is shown in FIG. 20. Latched post (2000) is shown having beveled latch (2002) pivotally disposed between post members (2006). Latched post (2000) is shown extending from backing (2004) of which only a portion is shown for clarity. Beveled latch (2002) is preferably integrally attached at a proximal end so that latch distal end (2010) is free to move. Beveled latch (2002) is also preferably beveled to provide a gripping surface once the device is secured in the patient. Because latch distal end (2010) may be free to move, latch (2002) may be configured so that latch distal end (2010) maybe biased to extend angularly away from post members (2006). As post (2000) is inserted into a patient's cranium, latch distal end (2010) may be urged towards post members (2006) to facilitate insertion by depressing lever (2008), located at the proximal end of latch (2008). Once latched post (2000) has been positioned in the patient, lever (2008) may then be released, thus allowing latch distal end (2010) to protrude angularly against the interior of the hole in the patient's cranium thereby providing a locking action.

A further variation of the post is shown in FIG. 21. Here, angled latch post (2100) is preferably an angled latch (2102) having a beveled surface and being integral with backing (2104) of which only a portion is shown for clarity. Angled latch (2102) may be integral with backing (2104) at the latch proximal end (2110) and disposed in-between post members (2106). Angled latch (2102) may further be biased so that the latch distal end (2112) is angled away from backing (2104) and protrudes from in-between post members (2106). Accordingly, as angled latch post (2100) is inserted into the patient's cranium, latch distal end (2112) may similarly be urged towards post members (2106) to likewise facilitate insertion. This movement or urging may be accomplished by depressing latch extension (2108), which may be integrally attached to both backing (2104) and angled latch (2102). Because latch extension (2108) may be attached in apposition to angled latch (2102), depressing it would thereby move latch distal end (2112) accordingly.

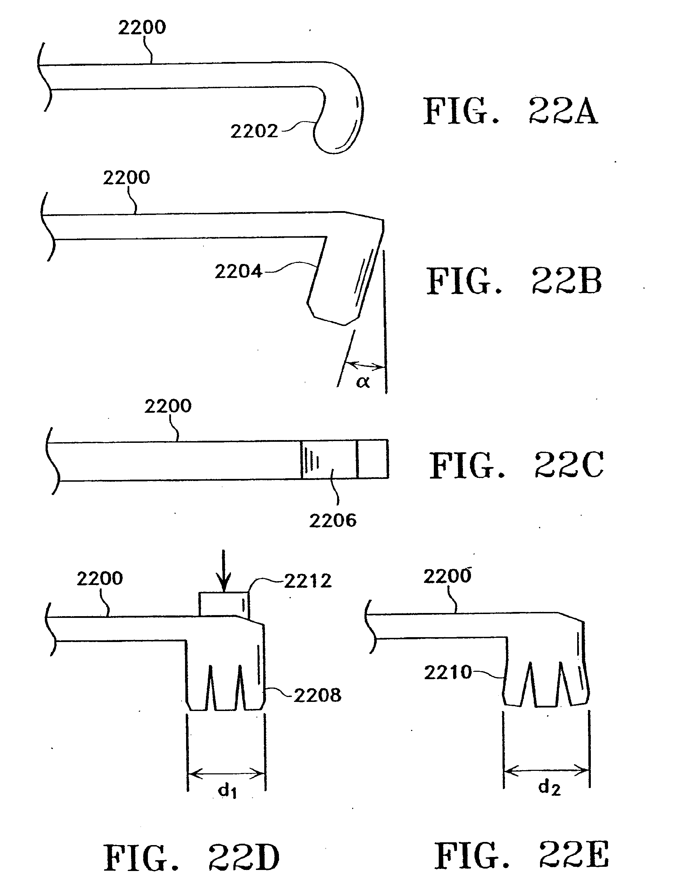

FIGS. 22A-22B show alternative variations of the post which may include any of the features discussed herein. FIG. 22A shows rounded post (2202) having a radiused distal end. FIG. 22B shows angled post (2204) which defines a predetermined angle, α, between a plane of backing (2200) and a longitudinal axis defined by angled post (2204). FIG. 22C shows another variation where a post is not used at all. Rather, a hole may be provided which has a diameter sufficient to receive a separate fastener. In this variation, the fastener may be used to secure backing (2200) to the patient's cranium through hole (2206). Fasteners may comprise any conventional fasteners, e.g., pins, nails, screws, and so forth. Alternatively, rather than securing the device via a fastener through a hole, the hole (2206) may be omitted entirely and the backing (2200) may be secured to the cranial surface via an adhesive, e.g., cyanoacrylate. Such an adhesive is preferably biocompatible and provides sufficient bonding strength to support the tissue or scalp when lifted.

FIGS. 22D-22E show an alternative variation where the post comprises radially expandable extensions. Expandable post (2208) is preferably integral with backing (2200) to provide a uniform device. FIG. 22D shows expandable post (2208) having a first diameter, d₁. This device may be inserted into the patient's cranium and positioned in a desired location and configuration. Once positioned, the diameter may be expanded by inserting expander device (2212), or using a tool configured to expand radially, which pushes against the inner surfaces of expandable post (2208). The resulting expanded configuration is shown in FIG. 22E where expanded post (2210) has a second diameter, d₂, which is larger than first diameter d₁ and thus aids in securing the device in place.

### Variations on Drilled Holes

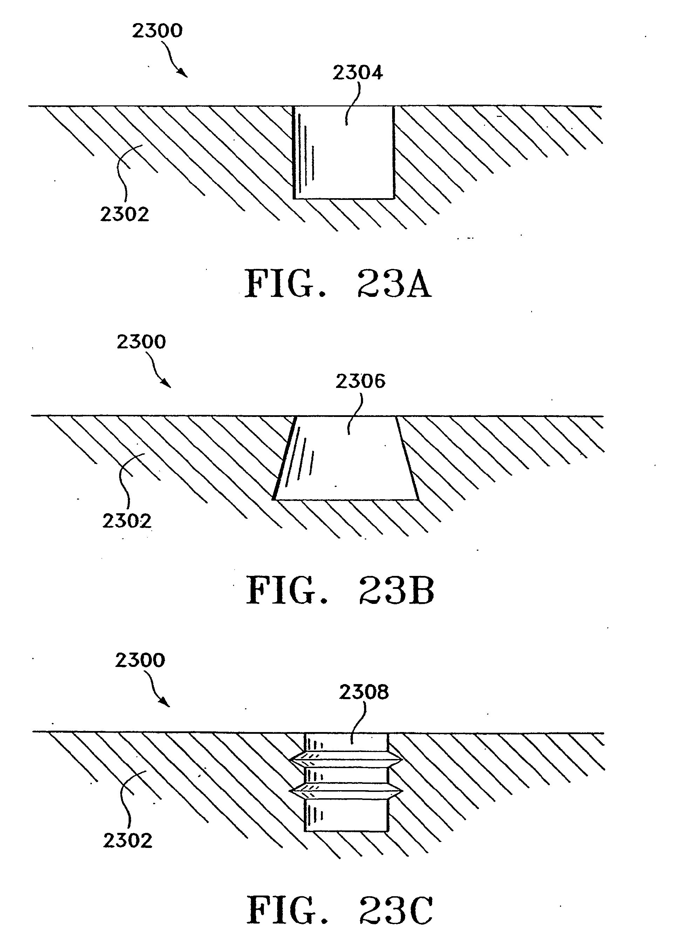

In anchoring a device within a patient, a hole may be drilled into the cranium or facial bone to receive a securing post of the device. As mentioned above, the hole may be drilled by any number of conventional drills or specialized surgical drills. FIG. 23A shows a cross-sectional view of a typical drilled hole (2304) in cranium (2300) which extends down into the cranial bone (2302). FIG. 23B shows another variation having angled hole (2306) which may be used to receive any of the post variations discussed herein. A further variation is shown in FIG. 23C where the hole may comprise keyed hole (2308). This variation shows keyed hole (2308) having two concentric grooves within the hole; however, any number of grooves or variations thereof may be incorporated depending upon the desired hole profile and the tightness of the fit of the post within the hole.

### Variations on Supportive Backings

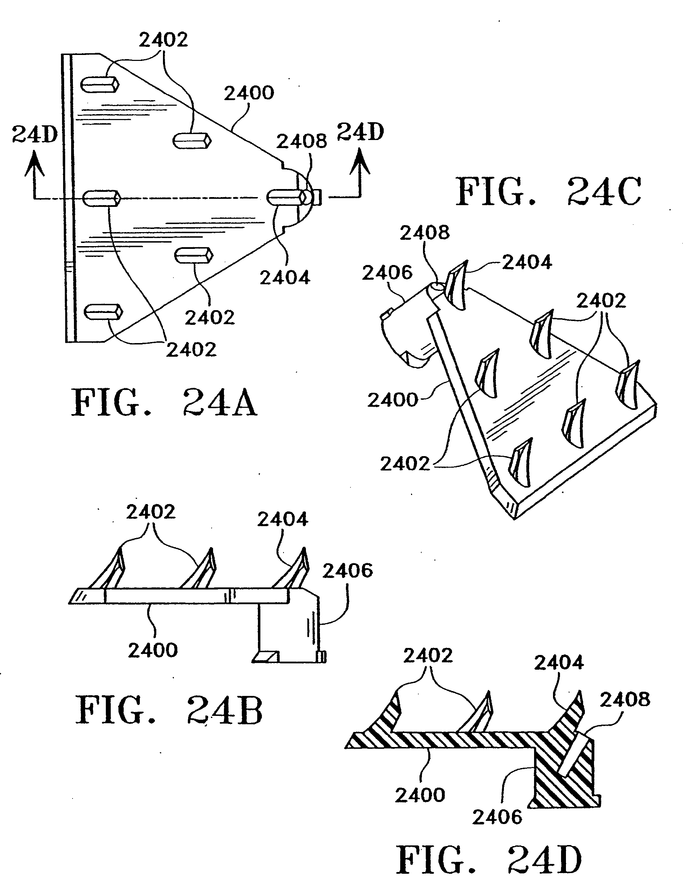

FIGS. 24A-24D show a variation on the device backing. FIGS. 24A-24B show a top and side view of a device which is similar in many aspects to the device as shown in FIGS. 13A-13C. The device comprises supportive backing (2400), post (2406), proximal cavity (2408), and attachment points (2402). However, this variation also comprises an additional leading attachment point (2404). This leading attachment point (2404) may be incorporated as a redundancy to ensure tissue adhesion should the other attachment points (2402) slip or tear from the scalp tissue. FIG. 24C shows a perspective view of the device with leading attachment point (2404). And FIG. 24D shows a view of cross-section 24D-24D from FIG. 24A. Proximal cavity (2408) is clearly seen to extend partially into post (2406); but post (2406) may incorporate other cavities and configurations as discussed above.

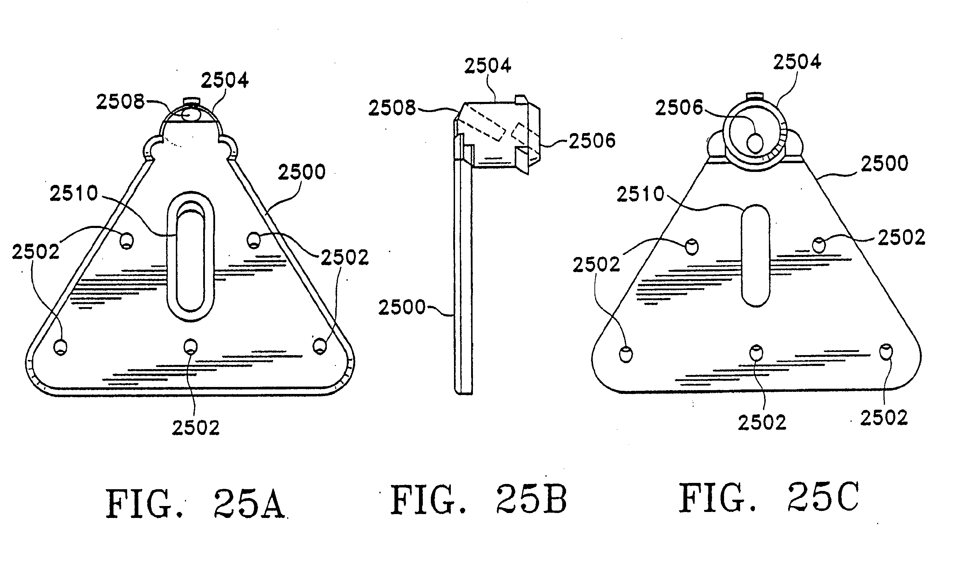

FIG. 25A shows a top view of supportive backing (2500). This variation is also similar in many aspects to the device as shown in FIGS. 13A-13C. The device may comprise post (2504), proximal cavity (2508), and through-hole (2510), which may be slotted or may comprise any other shape. Also, as seen in FIGS. 25B and 25C, the device may also comprise distal cavity (2506); however, this variation may have separatable attachment points which may be held in attachment point locations (2502). This variation may allow a doctor or surgeon to attach variously shaped attachment points in a variety of orientations relative to one another depending upon the desired result. Moreover, this variation may allow one to selectively attach attachment points at desired attachment point locations (2502). Any number of attachments points may be utilized; however, it is preferable that at least three attachment points or tines spaced relatively apart be used to optimize the holding capacity of the device to the tissue.

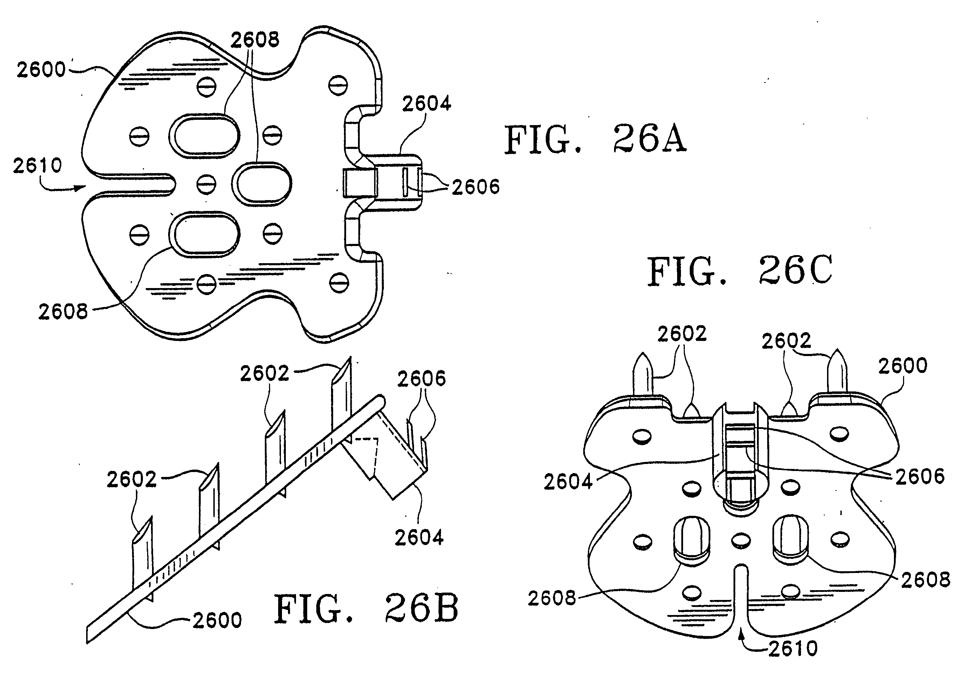

FIG. 26A shows a top view of an alternative variation for supportive backing (2600) which is configured to be flexible and hold multiple attachment points (2602). This particular variation may be configured to reduce the amount of material used and simultaneously increase the flexibility to allow backing (2600) to conform to the patient's cranium. Flexibility may be achieved via the use of through-holes (2608) and slot (2610) which are seen in FIGS. 26A and 26C. This variation also may incorporate post (2604) which may comprise anchoring tabs (2606), as seen in the side view of FIG. 26B, to aid in securing the device to the cranium.

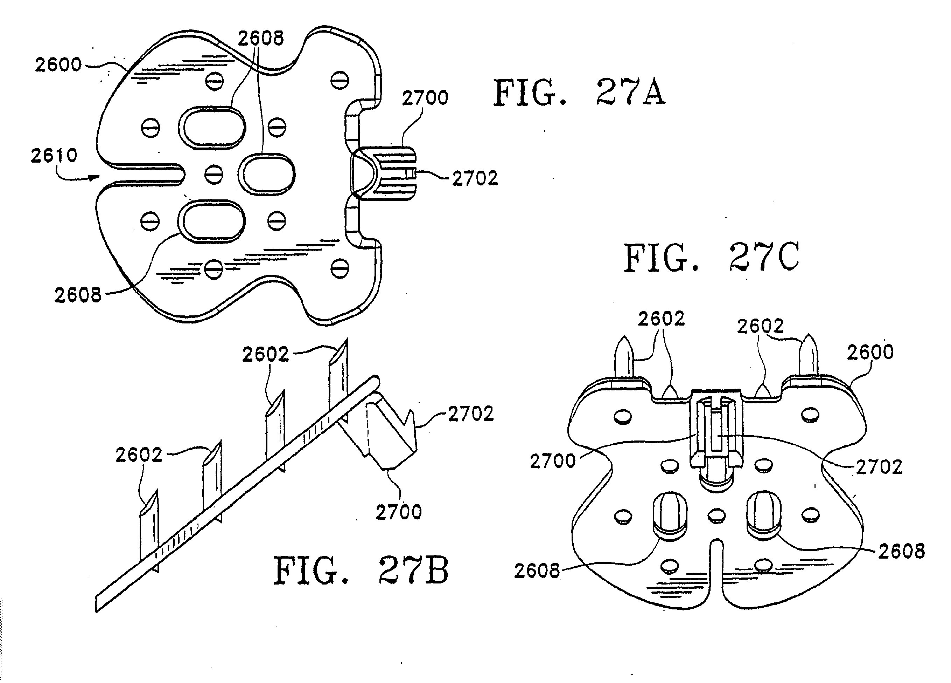

FIG. 27A shows a top view of another alternative variation for supportive backing (2600) which is similar in most aspects to the device shown in FIG. 26A. As seen in FIGS. 27A-27C, particularly 27B, this variation incorporates latched post (2700). Post (2700) may utilize a latching mechanism similar to the latched posts illustrated in FIGS. 20-21. This particular post comprises latch (2702) which is shown as having a hooked distal end.

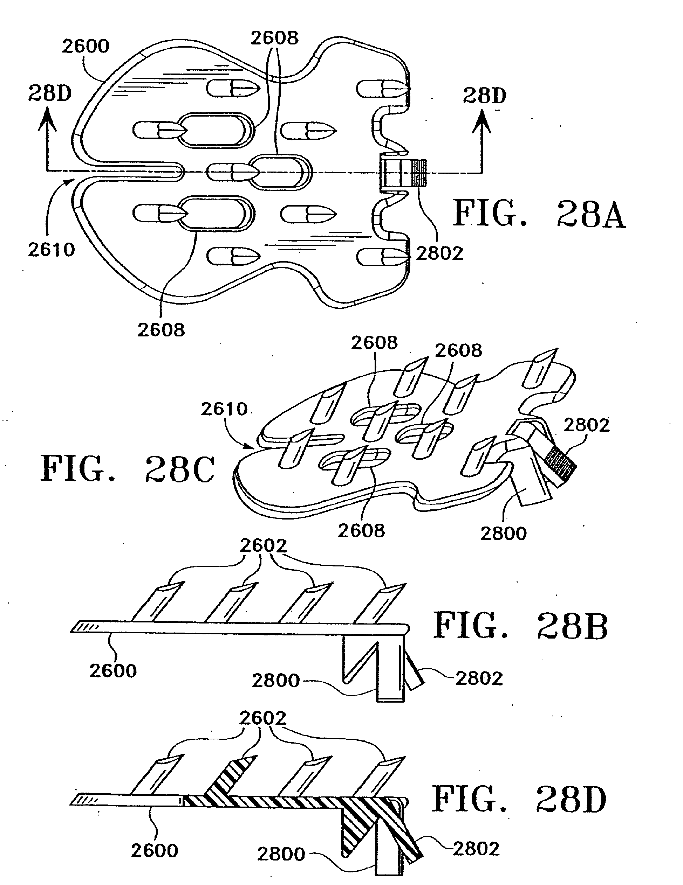

FIGS. 28A-28C shows top, side, and perspective views of a further variation for supportive backing (2600). This variation illustrates latched post (2800) having beveled latch (2802) which may be similar to the latching device shown in FIG. 21. FIG. 28D shows a view of cross-section 28D-28D taken from FIG. 28A. The latched post (2800) and the configuration of latch (2800) may be seen where latch (2802) is preferably integral with backing (2600).

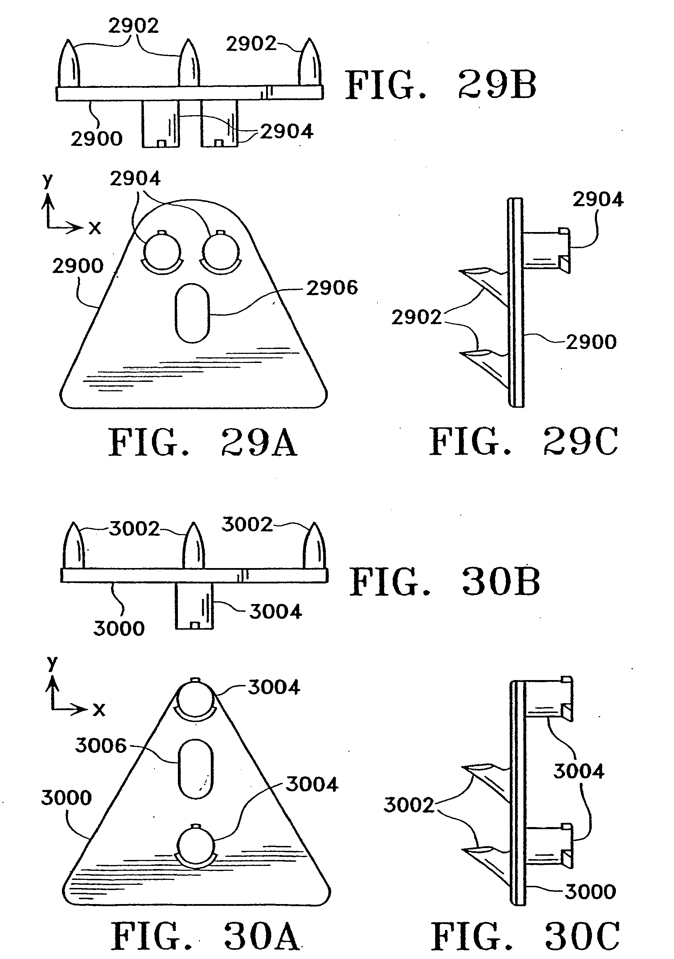

In addition to alternative backings, variations of devices having multiple anchoring regions may also be utilized. FIG. 29C shows a variation also having attachment points (2902) and through-hole (2906). As seen further in FIG. 29B, this variation may comprise a configuration where two posts (2904) are attached directly to backing (2900).

A further alternative backing having multiple posts is shown in FIG. 30A. Also seen in this variation are attachment points (3002) attached to backing (3000) and through-hole (3006) defined within backing (3000). However, this variation comprises two posts (3004), which are preferably integral with backing (3000), aligned along a y-axis. The additional post along the y-axis may aid greatly in also increasing the device resistance to rotation about posts (2904). This variation likewise may allow the device to be inserted at various angles within the cranium depending upon the desired results and the angle of desired lift. Furthermore, this particular variation may be desirable where cranial physiology would prevent two adjacent posts from being secured into the cranium.

### Placement Tools

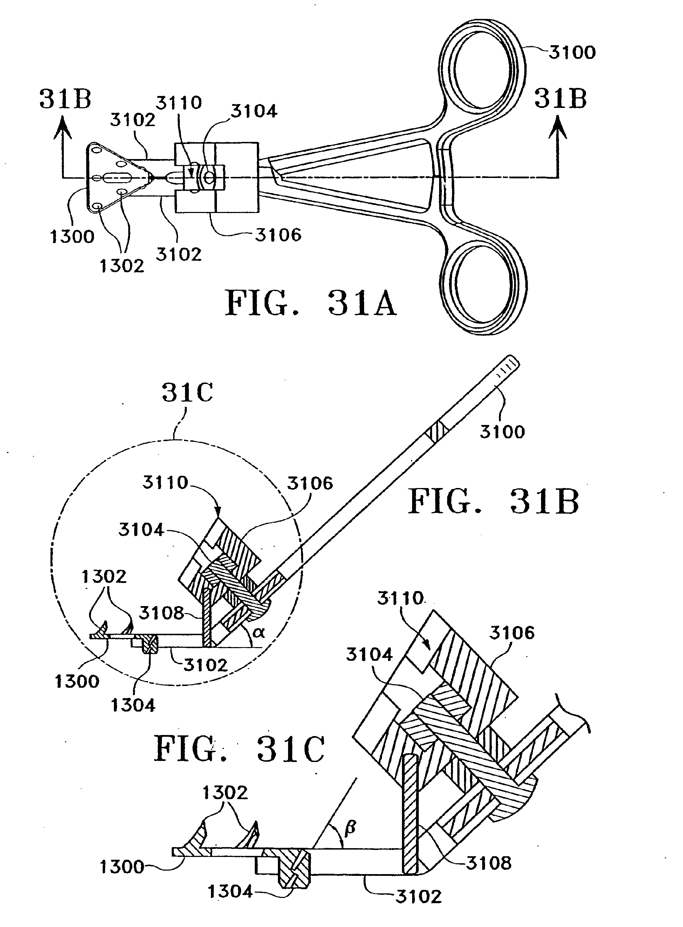

Many of the variations on the device may be inserted and secured into a patient in a number of ways. One such exemplary method involves using an insertion tool of a type shown in FIG. 31A. This variation shows a top view of such a tool which may serve several functions. This tool comprises manipulation handle (3100), by which a doctor or surgeon manipulates, for example, the device of FIGS. 13A-13C. As shown further in FIG. 31B, cross-section 31B-31B from FIG. 31A, handle (3100) may be hinged by any conventional methods but shown here as bolt hinge (3104). At a distal end of handle (3100) are grasping members (3102). These grasping members (3102) may generally be designed to have opposing members which may be urged together or apart, i.e., to close or open, as handle (3100) is urged about hinge (3104).

To prevent uncontrolled rotation of handle (3100) and to provide a way of securely grasping the device, handle (3100) may also comprise a locking mechanism which may hold handle (3100) and grasping members (3102) in a desired position. Grasping members (3102) are preferably designed or configured to securely hold the supportive backing (1300) relatively planar with grasping members (3102) such that attachment points (1302) face away from the patient during insertion. It is further preferable that grasping members (3102) securely hold the device via anchoring post (1304) to allow easy handling and insertion. As seen in FIG. 31B, grasping members (3102) are preferably angled relative to a plane defined by handle (3100) at a predetermined angle, α, to further allow easy insertion of the device.

FIG. 31C shows a close-up cross-sectional view of the distal end of the insertion tool. As shown, also attached to hinge (3104) is support block (3106). Support block (3106) is preferably configured to attach to handle (3100) at hinge (3104) yet still allow rotational movement of the tool about hinge (3104). Support block (3106) also preferably defines channel (3110) through a top surface of support block (3106), as shown in FIGS. 31A-31C. Channel (3110) may run substantially parallel relative to a symmetrical axis defined by the insertion tool. Support block (3106) may be supported by support post (3108) which may help in preventing rotation of support block (3106) about hinge (3104) as well as maintaining a position of the block relative to handle (3100).

Further seen in FIG. 31C, channel (3110) in support block (3106) is preferably angled relative to the plane defined by handle (3100). While grasping members (3102) are angled at an angle, α, relative to handle (3100), channel (3110) may be angled relative to grasping members (3102) at a desired angle, β. This angle β is preferably similar to the angle formed by attachment points (1302) relative to supportive backing (1300). Angling channel (3110) may allow a mating block, described below in further detail, to run along channel (3110) and press against the tissue to be lifted against attachment points (1302). A block pressing against tissue to be set on attachment points (1302) allows for optimal piercing of the tissue if the force applied by the block is in the same or similar angle or direction as attachment points (1302).

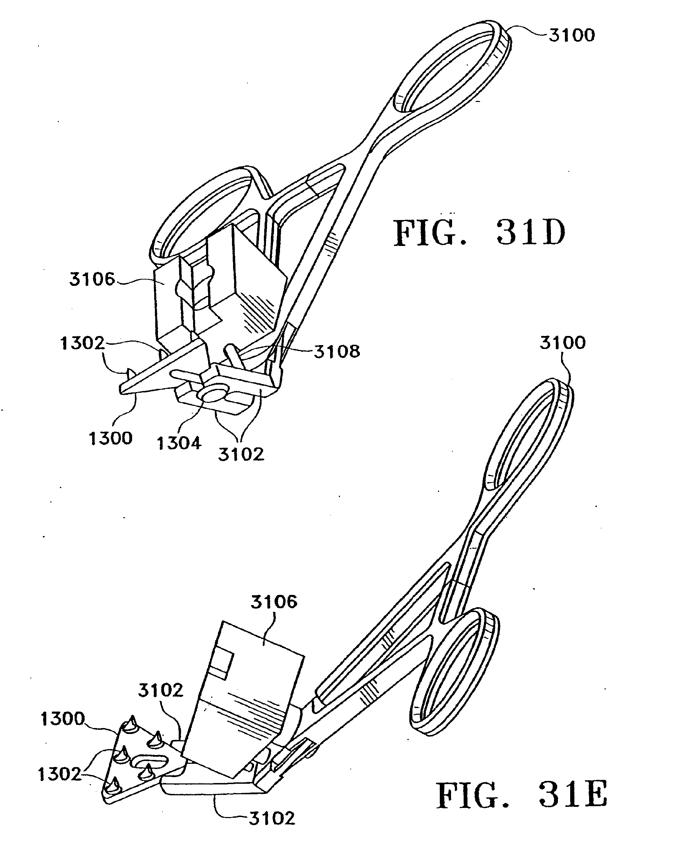

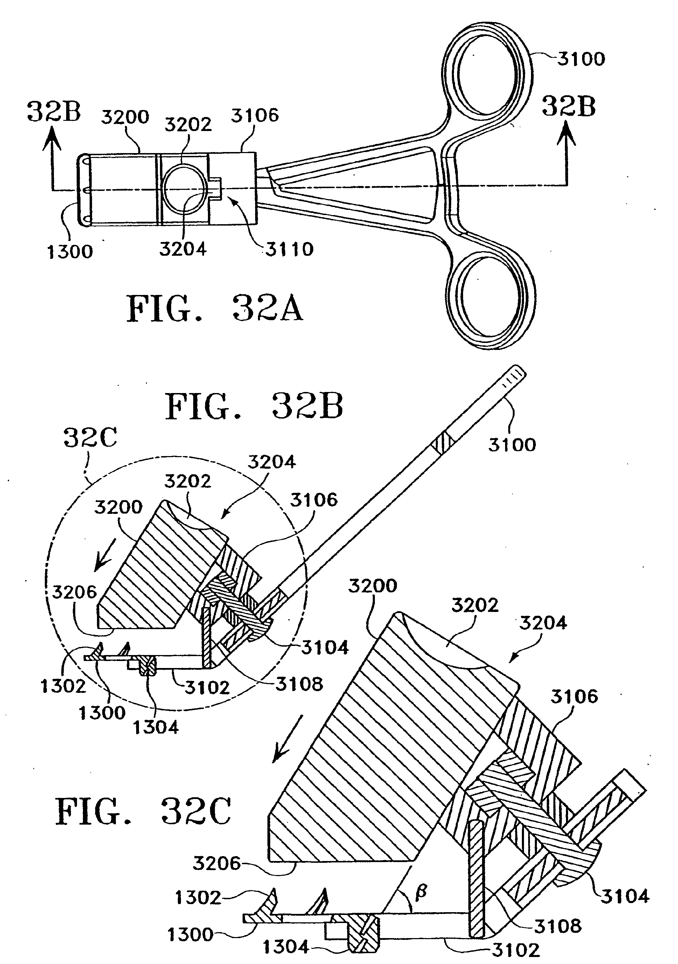

FIGS. 31D and 31E show a bottom and a top perspective view, respectively, of the insertion tool from FIG. 31A grasping an device. As seen in FIG. 32A, the same insertion tool from FIG. 31A is shown with the addition of depressible block (3200) mated with support block (3106). Depressible block (3200) may be mated with support block (3106) via channel (3110), into which mating slide (3204) may be inserted. Slide (3204) may be an integral extension of depressible block (3200) and is preferably configured to allow a degree of tolerance relative to channel (3110) so that depressible block (3200) may slide freely or when urged via channel (3110) and mating slide (3204), as shown by the arrow in FIG. 32B.

FIG. 32B also shows a cross-section 32B-32B from FIG. 32A. Depressible block (3200) further illustrates depression region (3202), which may be a slight indentation defined in the surface facing away from the patient during insertion. Depression region (3202) may serve as a locator for the optimal region the physician may depress to force depressible block (3200) and contact surface (3206) downward against the tissue and attachment points (1302) in order to set, or pierce, the tissue. FIG. 32C shows a close-up cross-sectional view of the distal end of the insertion tool with depression block (3200) inserted. Contact surface (3206) is the surface which ultimately presses the tissue against attachment points (1302) and is preferably relatively parallel with the plane defined by grasping members (3102) and supportive backing (1300) to present the greatest surface area pressing against the tissue. Depressible block (3200) is further preferably configured to slide or run along the same angle, β, at which support block (3106) is set to provide a planar contact surface (3206) to press against the tissue at an optimal angle, which may be at the same or similar angle as attachment points (1302), as discussed above.

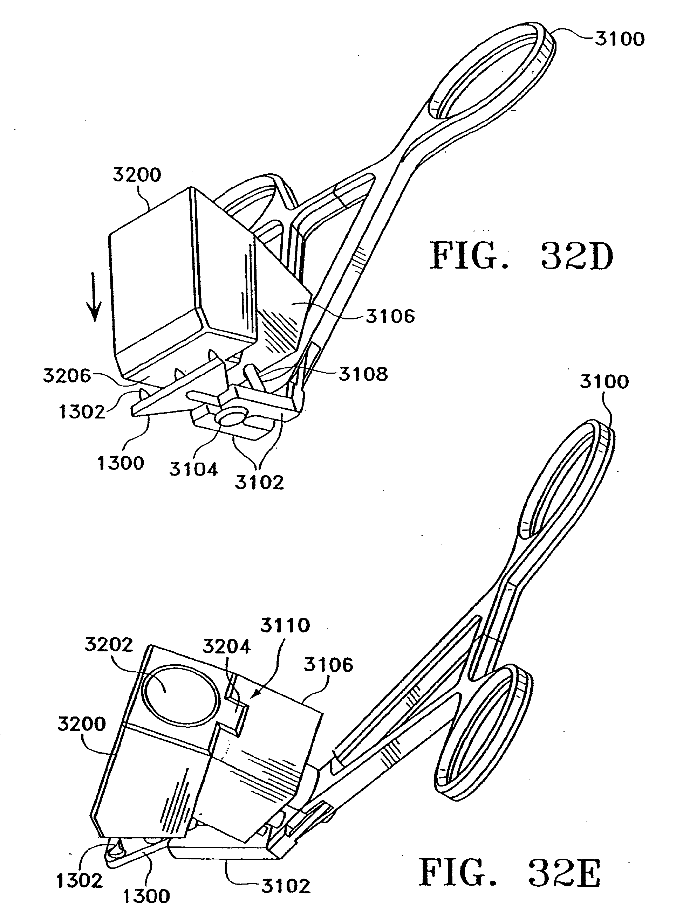

FIGS. 32D and 32E show a bottom and a top perspective view, respectively, of the insertion tool from FIG. 32A with depressible block (3200) set in channel (3110). Although the placement tool has been described with depressible block (3200), the tool may also be used without a block for depressing the tissue or scalp against the attachment points (1302). Rather, affixing or setting the tissue may also be done by hand, i.e., simply depressing the tissue with the hand and fingers against attachment points (1302).

### Orbital Fracture Procedures

Another variation of the present disclosure includes approximation of soft tissue in orbital fracture repair and other craniofacial and maxillofacial surgical procedures. One variation of the present disclosure features a supportive backing which is secured to a fracture site via fasteners such as screws. The supportive backing or plate set fragmented bones. The present disclosure also includes a plurality of attachment points which extend from the supportive backing such that soft tissue may be conveniently suspended on the attachment points. Examples of attachment points include tines.

Notably, the present disclosure eliminates the use of sutures to fixate soft tissue to the underlying fracture site. Consequently, typical problems associated with suturing soft tissue to the underlying bone are eliminated.

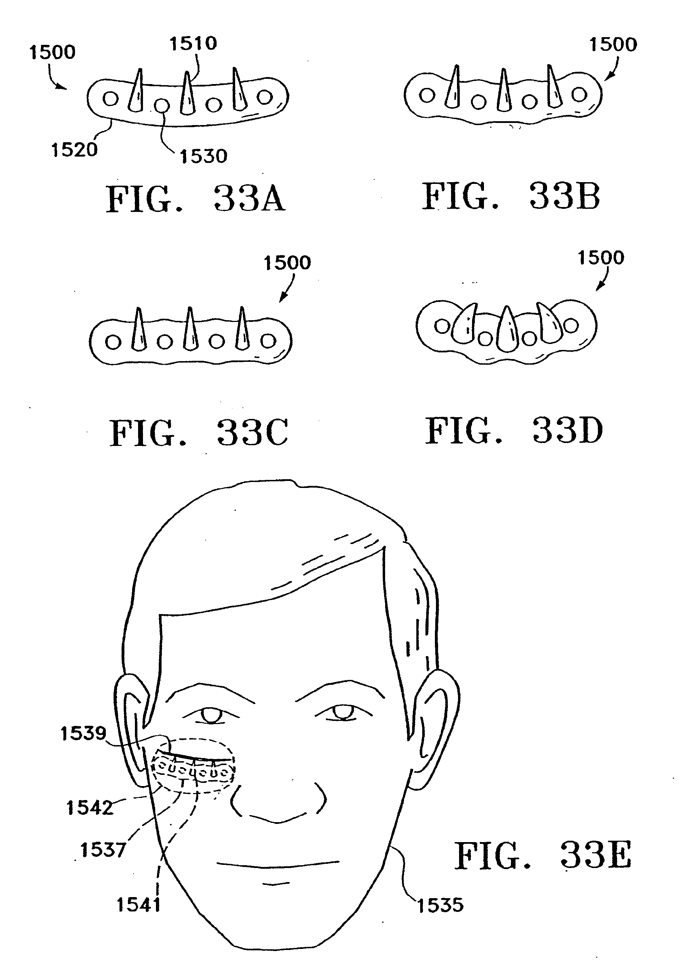

The present disclosure includes various shapes which are useful in approximation of soft tissue in orbital fracture repair and other craniofacial and maxillofacial surgical procedures. A preferred set of shapes is illustrated in FIGS. 33A to 33D. FIGS. 33A to 33D are front views of a tissue approximation device (1500) suitable for use in orbital fracture reconstruction procedures. As shown in FIG. 33A, attachment points (1510) extend from backing (1520).

The tissue approximation device (1500) also features a number of through-holes (1530). The through-holes provide an opening for receiving a fastener such as a pin or screw. The holes (1530) may be equally spaced or unequally spaced along the backing (1520). There may be one or more holes (1530).

In addition to the shapes shown in FIGS. 33A-33D, the plate or supportive backing may be shaped as a character such as but not limited to C, H, I, L, T, U, V, Λ, and ∩. The supportive backing may also be curved away from the direction of the tines or curved in a direction orthogonal to the direction of the tines. The supportive backing may also be convex or concave when viewed from the front or the side (not shown).

Except where stated otherwise, the characteristics of the attachment points (1510) and supportive backing (1520) are similar to the attachment points and backings described in the variations set forth above. For example, the supportive backing is preferably fabricated from biocompatible materials, biodegradable materials, or materials which are generally absorbable by the patient. The device may also be made from biological materials.

The device may further contain bioactive compounds or therapeutic agents. Such agents may be impregnated in the device, coated on the device, sprayed, or otherwise deposited on the device. Multiple coatings may be applied to delay release of such agents. Suitable agents include proteins, pharmaceuticals, genetic material, and other chemicals or compounds which have a useful effect in humans. Other non-limiting examples of agents include hydroxyapatites, tricalcium phosphates, bone growth factors, and bone morphogenic proteins.

The device may also be made of a material and thickness such that it may be shaped intra-operatively to the patient's anatomy by applying heat to the device. Such devices are well suited for orbital reconstruction and suspensions where curves are desirable to accommodate facial bones.

An illustration of an exemplary application is shown in FIG. 33E. FIG. 33E shows a head (1535) with a tissue approximation device (1537) secured to an orbitalfacial fracture site (1542) underneath wound (1539). The device (1537) is shown as a rigid plate and is useful in setting fragmented bones.

Characteristics of the supportive backing of device (1537) will depend on its application. In this illustration, where bone setting is required, the backing must be generally rigid and have a sufficient thickness to fasten the bone fragments together. In other applications, however, where the device is used for tissue approximation and no bone setting is required, the backing may be less rigid, less thick, and more conforming.

FIG. 33E also shows the lower portion of wound (1539) set or suspended on tines (1541). In this manner, the soft tissue covering the device (1537) remains suspended and there is no need for additional sutures to attach the soft tissue to the underlying bone. There is also no need for any additional steps to suspend the soft tissue.

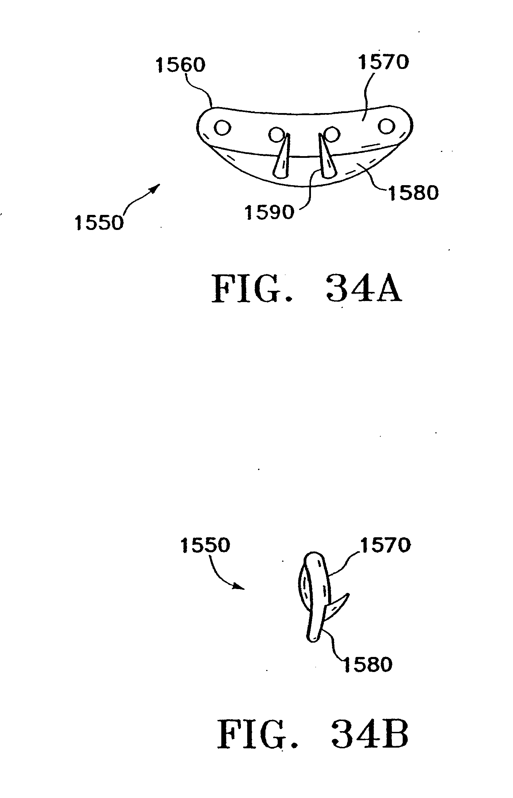

FIGS. 34A and 34B show a variation which is also useful in orbital reconstruction procedures. In particular, FIG. 34A shows a tissue approximation device (1550) having a supportive backing (1560) divided into two discrete regions. The first or plate region (1570) includes several through-holes and is "tineless." That is, no tines or attachment points are shown in the plate region (1570) of FIG. 34A. The second or tine region (1580) features two tines (1590) to serve the function as indicated in the above described variations. The plate region and tine region may be separate structures joined together or they may be integral with one another.

FIG. 34B shows a side view of the tissue approximation device having a variation in thickness. In particular, the tine region (1580) is thinner than the plate region (1570). Such a device is suitable for applications requiring a thicker substrate in one bone location. In orbital and maxilla fractures, for example, devices with a varying thickness can be useful. The tine region may be thicker than the plate region (not shown).

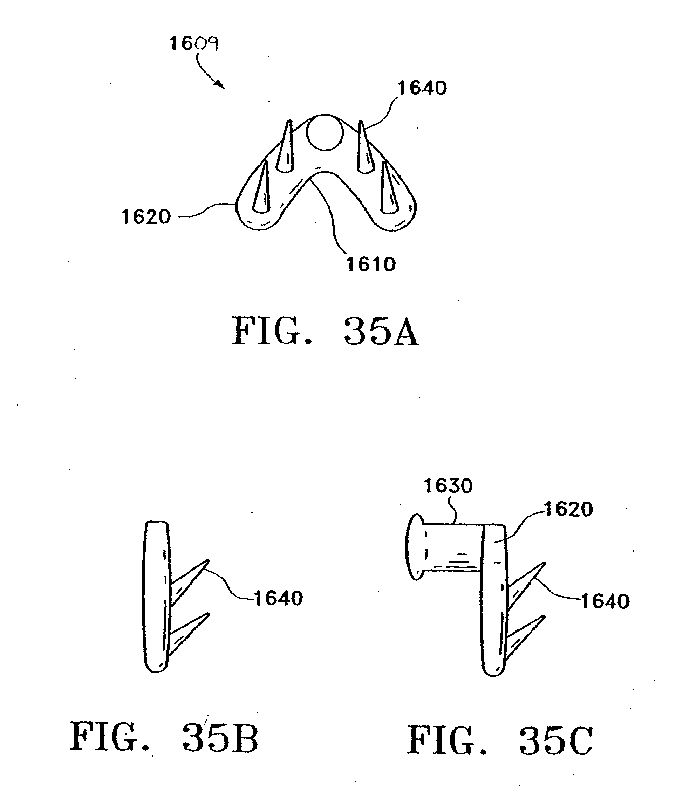

FIGS. 35A to 35C illustrate another exemplary variation. In particular, FIG. 35A shows a tissue approximation device (1609) in a horseshoe shape. Arc (1610) is provided to avoid covering nerves such as the infraorbital nerve in, for example, midface lift procedures. While a horseshoe shape is shown in FIG. 35A, other shapes having arcs, slots, or curves may be contemplated which avoid covering nerves or other anatomical structures which are desirably left uncovered.

As shown in FIG. 35C, backing (1620) may have an anchoring post (1630) extending therefrom to secure the device in bone. The anchoring post (1630) may eliminate the need for separate fasteners. FIGS. 35A to 35C also feature four tines symmetrically disposed on backing (1620). The tines serve the same function as described in the preceding exemplary variations.

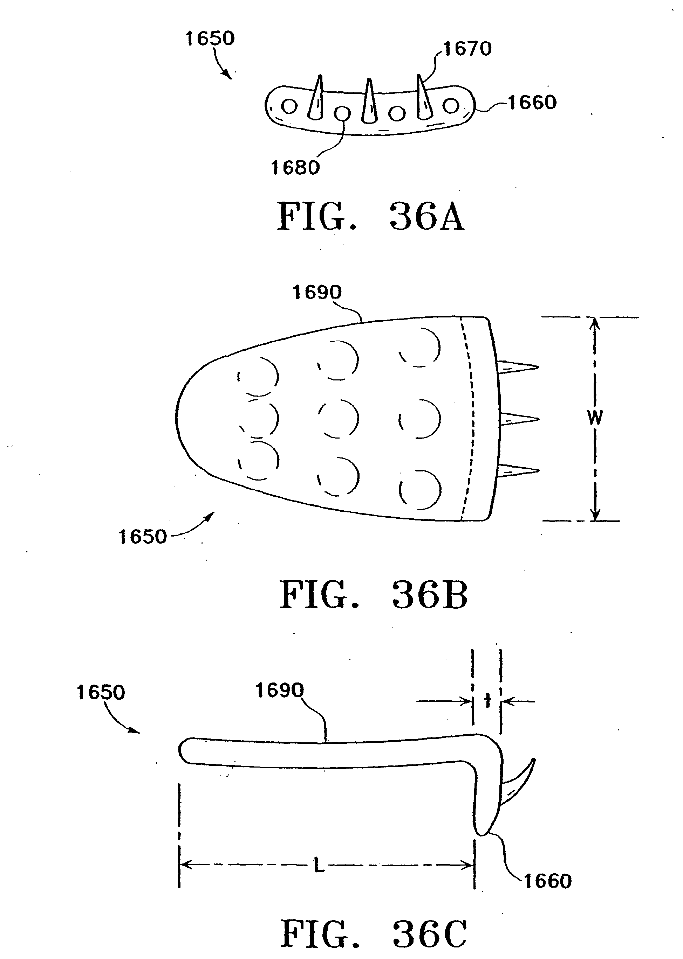

FIGS. 36A to 36C illustrate another exemplary variation useful in orbital rim and orbital floor reconstruction. As shown in FIGS. 36A to 36C, the device (1650) includes a backing (1660), tines (1670), and through-holes (1680) similar to the variations described above. However, device (1650) features a floor (1690) perpendicularly extending from backing (1660). The floor (1690) is shown as substantially flat and has a width approximately equal to the width of the plate or backing (1660). Preferably, the width W of the backing (1660) is sized equal to or less than the width of the orbit. The thickness t of backing (1660) is preferably in the range of 0.1 to 5 mm and more preferably between 0.5 to 2.5 mm. The length L of the floor (1690) is limited also by the depth of the orbit and the thickness of the floor is preferably in the range of 0.3 to 1 mm.

The device shown in FIGS. 36A to 36C is particularly suitable in severe orbital fractures that include fractures of the orbital floor where additional support is required. That is to say, a floor (1690) is suitable in repairing severely damaged sites where the orbit bones are fragmented and fixation is needed in multiple dimensions. The floor may have other shapes and may be adapted to particular sites and depths as appropriate for the severity and type of fracture.

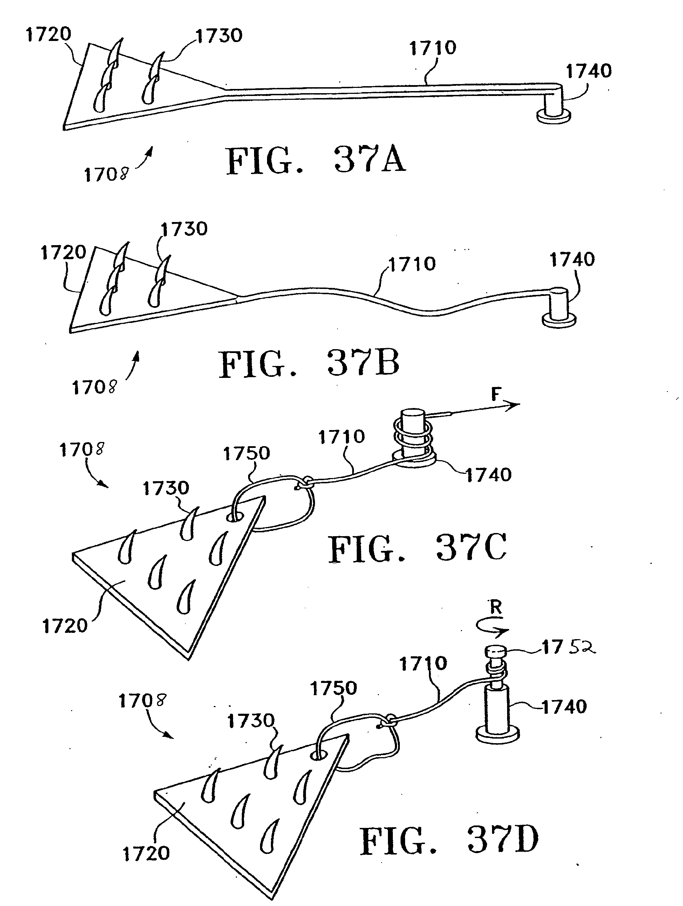

FIGS. 37A to 37D illustrate another exemplary variation. FIGS. 37A to 37D show a tissue approximation device (1708) with an extension member (1710) extending from supportive backing (1720). Similar to the variations described above, backing (1720) includes one or more tines (1730) for suspending soft tissue such as cheek tissue in the orbital region. Unlike the previous variations, however, an anchor post (1740) is separated from backing (1720) by extension member (1710).

This variation is suitable for procedures where the preferred anchoring position is not adjacent the soft tissue to be suspended. The exemplary variation thus provides for the suspension of soft tissue remote or distal to an anchoring position. While only one plate is shown attached to anchor (1740), the exemplary variation also encompasses multiple plates attached to a single post.

Extension member (1710) may be either solid or flexible (such as a tether) as shown in FIGS. 37A and 37B respectively. If solid, a surgeon may be provided with a number of devices having varying lengths. A device having a solid extension member may be used to indirectly suspend soft tissue above or below the anchor post (1740).

Soft or threadlike extension members may be suitable for indirectly suspending soft tissue below the anchoring position. Advantageously, the length of soft or threadlike extension members may be adjusted and varied during a surgical procedure.

For example, FIG. 37C shows a flexible extension member (1710) being manipulated by a force F which decreases the distance between the backing (1720) and post (1740). In this manner, a post may be secured to a bone site and the backing or plate (1720) may be positioned a selected distance from the anchor (1740).

Another variation is shown in FIG. 37D. In FIG. 37D, the extension member (1710) is adjusted by rotating a knob (1752) to wind the extension member around the knob thereby decreasing the distance between the post (1740) and the plate or backing (1720).

The extension member may be joined to the plate or backing (1720) in a number of ways including a suture (1750), adhesive, a knot, an ultrasonic weld, a pressure fit, or any other suitable joining technique.

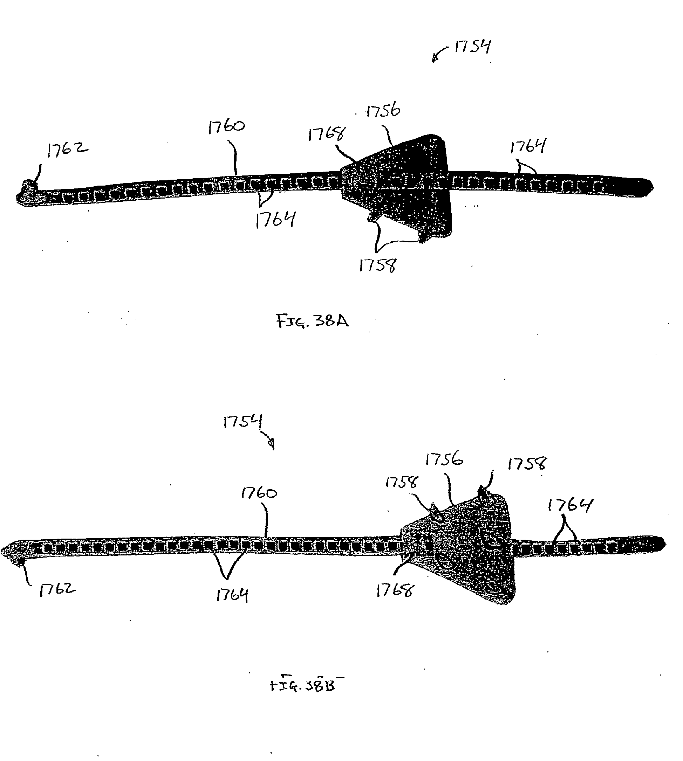

Another variation similar to that above may be seen in FIGS. 38A and 38B, which show two views of an adjustable tissue approximation device (1754). This remote anchor variation may have particular mid-face applications. A supportive backing (1756) is shown with one or more tines (1758) extending from the backing (1756) in a manner described above. Backing (1756) may be slidingly connected by an adjustable leash or extension member (1760) to an anchor or post (1762). The adjustable leash (1760) maybe made with multiple engagement holes (1764) defined along its length for adjustably engaging backing (1756) selectively along the length of the leash (1760). Backing (1756) and tines (1758) may be made according to any of the variations as described above. For example, the backing may be triangularly shaped, as shown in this variation. Alternatively, the backing may also be shaped in a variety of other configurations, e.g., rectangles, squares, circles, linear members, or any of the other configurations described above.

This particular variation may be used for surgical repositioning and suspension of the infraorbital mid-face of a patient. The device (1754) may be deployed and positioned beneath the patient's mid-face through remote incisions, e.g., buccal (oral), eyelid (subciliary or transconjunctival), or temporal incisions. Anchor (1762) may be positioned securely within a drilled hole located in the zygomatic bone, e.g., into the infraorbital rim or medial zygomatic arch. Backing (1756), while attached to anchor (1762) via leash (1760), may be positioned below the infraorbital rim of the midface. Depending upon the patient's physical characteristics and mid-face geometry, the backing (1756) may be positioned adjustably along leash (1760) and locked in place by engagement with locking holes (1764) once desirably placed. Accordingly, the leash (1760) may have an adjustable length ranging, for example, anywhere between 1.27 to 2.54 centimetres (0.5 to 5 inches) in length. The tines (1758) which extend from backing (1756) are adapted to protrude into the mid-face tissue and approximate the tissue while optionally adjusting the position of backing (1756) along the length of leash (1760).

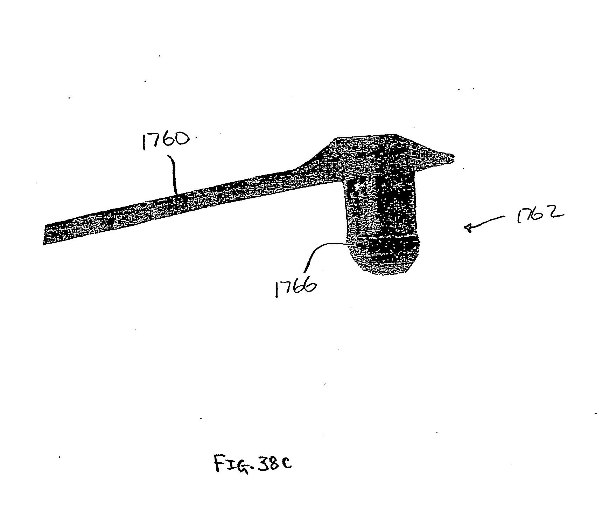

FIG. 38C shows a side view of a variation of anchor (1762). As shown, anchor (1762) may have an enlarged diameter (1766) distally located along anchor (1762) to facilitate secure engagement within the bone hole. Anchor (1762) may also be shaped or configured in any of the other variations described above for the post so long as it provides for a secure attachment to the bone to resist being pulled out, e.g., the post may be configured as an interference post so that it is secured via an interference fit within the bone, the post may also be threaded to allow for threaded engagement within the bone hole, or any of the other post variations described above. Anchor (1762) may further be positioned to form an acute angle relative to leash (1760), as shown in the FIG. 38C, to aid in securing backing (1756) and anchor (1762) in position once deployed.

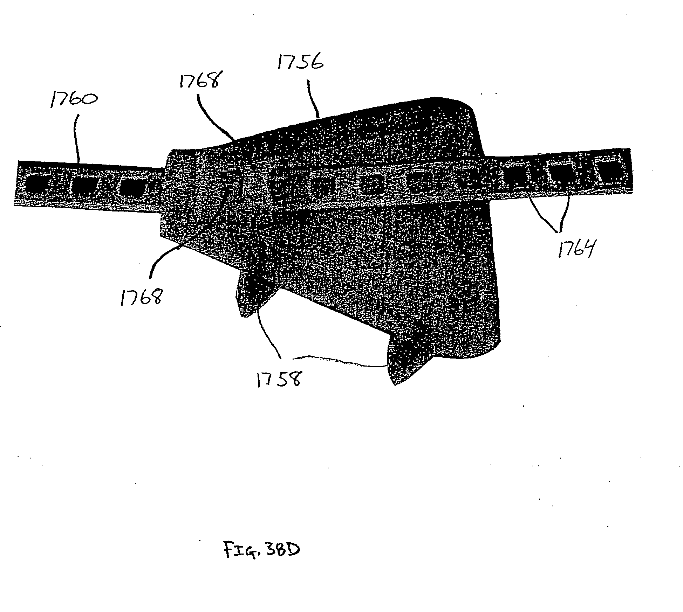

FIG. 38D shows a close-up view of leash (1760) adjustably secured through backing (1756). As shown, leash (1760) may extend through an adjustable latch (1768) formed on a proximal end of backing (1756). Latch (1768) may be formed on either side of backing (1756), i.e., on the same side as tines (1758) or on the opposite side of backing (1756). To securely engage leash (1760), latch (1768) may have a pawl (1770) attached within the latch (1768) which may be articulated to releasably engage holes (1764). This configuration allows for the multiple release and tightening of backing (1756) relative to leash (1760) during deployment and for post-operative adjustments, if necessary.

Pawl (1770) may also be configured into a uni-directional pawl, such as a zip-tie, which would allow travel of the leash (1760) only along a single direction relative to the backing (1756). Furthermore, leash (1760) is preferably configured to have a low profile against the tissue to remain non-obtrusive. Leash (1760) is further preferably configured to withstand tensile loads which may be generated by the approximated tissue. For instance, the device (1754) may be configured to withstand tensile loads along the leash (1760) of up to, e.g., 7 lbs, and for post-operative tensile loads of, e.g., 1,37 kg (3 lbs) for about 24 hours, and then, e.g., 0,68 kg (1.5 lbs) for several more days post deployment.

Alternatively, the leash may be configured to engage with the backing in a number of different ways. The leash may also be configured to releasably engage with the backing through the use of protuberances, e.g., nubs, bumps, etc., located along the length of the leash. These protuberances may be adapted to interlock with a corresponding locking arm located on the backing. Moreover, aside from protuberances, other methods such as notches, indentations, etc., may also be defined along the leash. Essentially, any known variety of releasable engagement methods as known in the art may be used accordingly on the leash to accomplish adjustability relative to the backing.

Backing (1756) is preferably configured to be non-obtrusive through the skin of the patient; it may therefore have a thickness ranging from, e.g., 0.5 to 1 mm (about 0.02 to 0.04 in.). The entire device (1754) or portions of it may also be made entirely of any of the bioabsorbable materials described above provided the structural strength is sufficient to withstand the tensile loads.

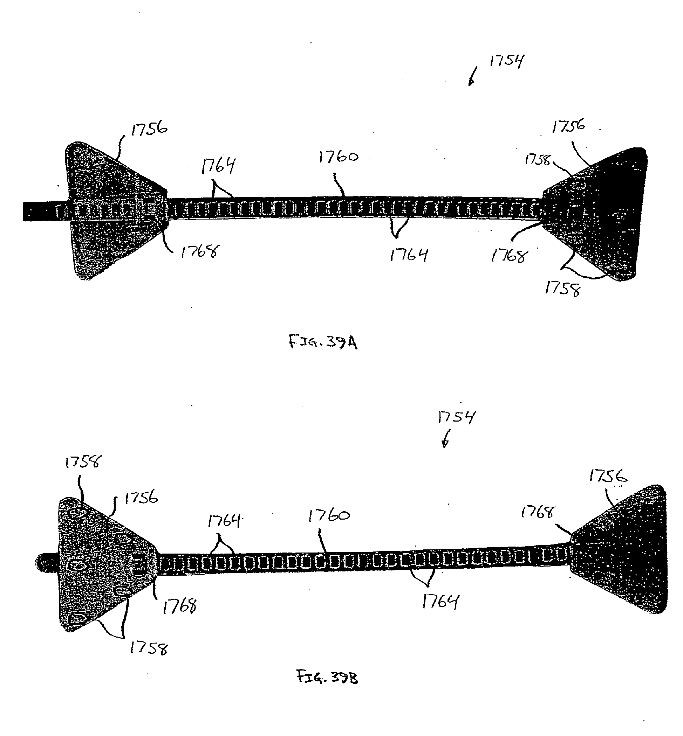

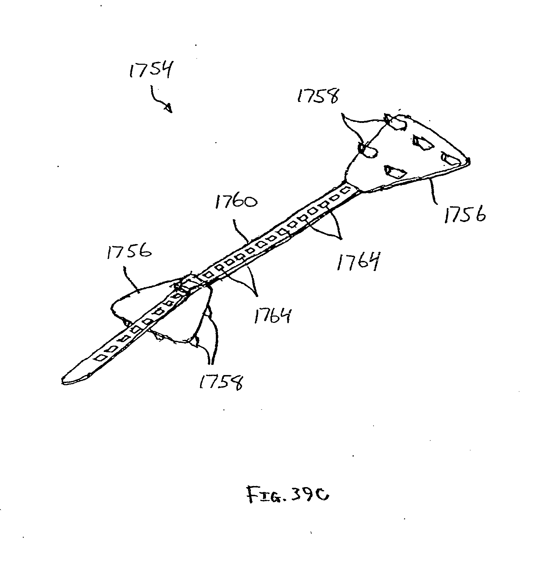

Yet another variation is seen in FIGS. 39A to 39C, where device (1754) includes a pair of tined backings (1756) slidably attached along leash (1760). Both backings (1756) can face the same way, or be inverted relative to one another so the tines (1758) from one backing face in the opposite direction compared to the other (as shown in FIG. 39C) for anchoring the device to soft tissue rather than to bone. This variation may be used likewise for surgical repositioning and suspension of the mid-face by affixing the device (1754) from, e.g., the mid-face, to, e.g., the temporalis fascia. The pair of supportive, tined backings (1756) can have the same or different size and configuration depending upon the desired results and the particular physiology of the patient.

As shown, leash (1760) may be configured to have multiple locking holes (1764) defined along the length of the leash (1760). Moreover, one end of the leash (1760) may be non-adjustably attached to or integrally formed with one of the backings (1756), leaving the other backing releasably adjustable along leash (1760). Alternatively, both backings (1756) may be adjustably positionable along leash (1760). Another alternative is to have both backings (1756) non-adjustably attached at the ends of leash (1760).

When deployed, the tines of either backing may be attached into, e.g., the temporalis fascia, such that the tines protrude into the deep tissue of the temporalis fasica and the muscle rather than into the scalp. The remaining backing may then be secured into the mid-face tissue, preferably in both sub- and supra-periosteal dissections. One or both backings, depending upon the configuration, may be adjusted along the leash before or after placement into the tissue to adjust for the desire amount of tissue suspension.

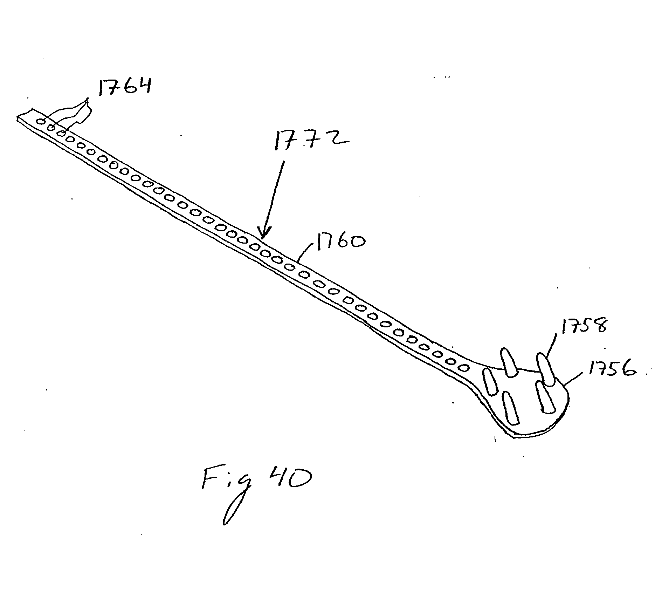

FIG. 40 illustrates approximation device (1772), a variation of approximation device (1754), where backing (1756) is integrally formed with or attached to leash (1760), with one or more tines (1758) extending from the backing (1756) preferably in a non-orthogonal manner (e.g. preferably angled toward leash (1760)). One or more of the engagement holes (1764) on leash (1760) are directly used to anchor the tissue approximation device (1772) in place after deployment using sutures, screws, staples, etc., with any excessive length of leash (1760) preferably being cut away beyond the anchoring position. Tissue approximation device (1772) of this configuration is ideal for mid-face tissue approximation, with or without a delivery device, as described next.

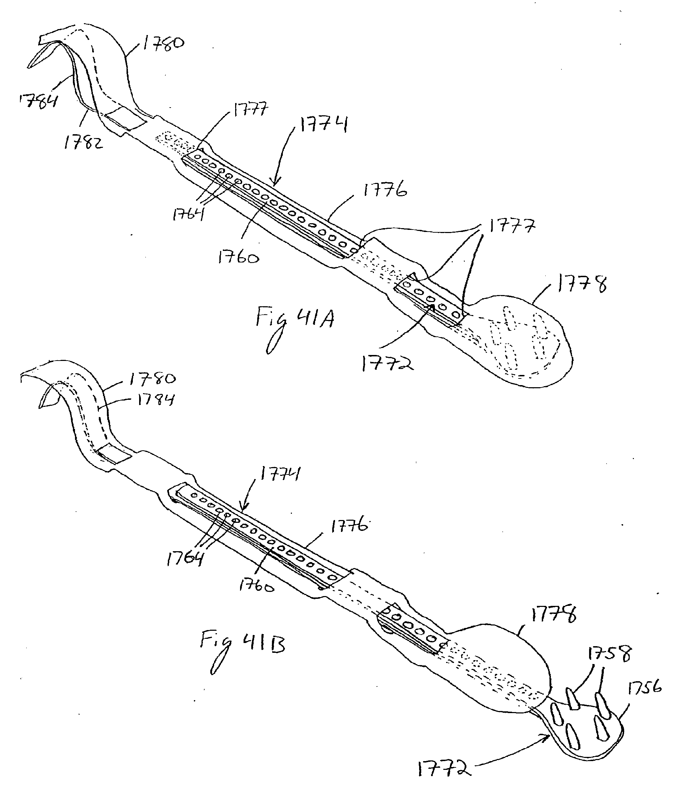

FIGS. 41A/B illustrate an optional delivery device (1774) for the tissue approximation device (1772) shown in FIG. 40. Delivery device (1774) includes an elongated support member (1776) that terminates at one end in a protective cup (1778) and in a shaped handle (1780) at the other end. Elongated support member (1776) includes a plurality of slots (1777) through which the leash (1760) slides. In its insertion position, the tines (1758) are contained within cup (1778), as shown in FIG. 41A. The delivery device (1774) also includes an implementation member (1782) that is disposed underneath and attached to or abutting tissue approximation device (1772). When a handle portion (1784) of implementation member (1782) is pressed toward handle (1780), the elongated support member (1776) is slid back to retract cup (1778) from over backing (1756) and tines (1758), leaving tines (1758) exposed as shown in FIG. 41B. Elongated support member (1776) is preferably made of plastic, and implementation member (1782) is preferably made of stainless steel.

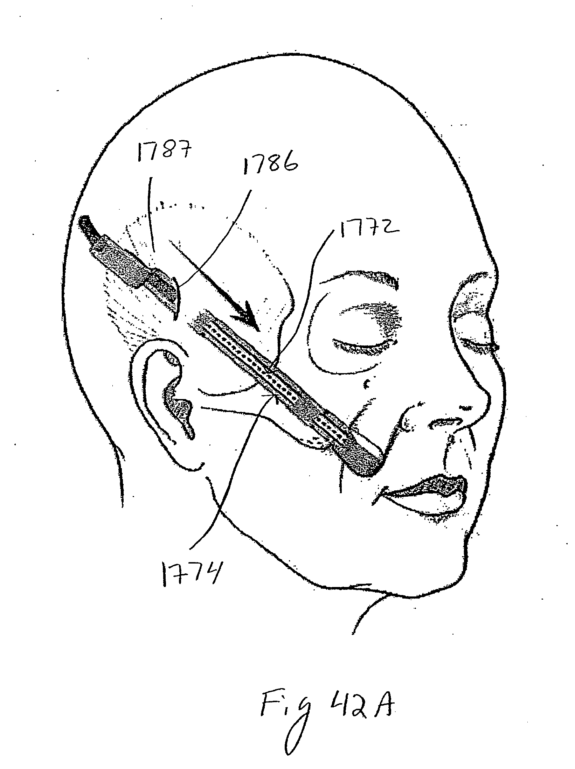

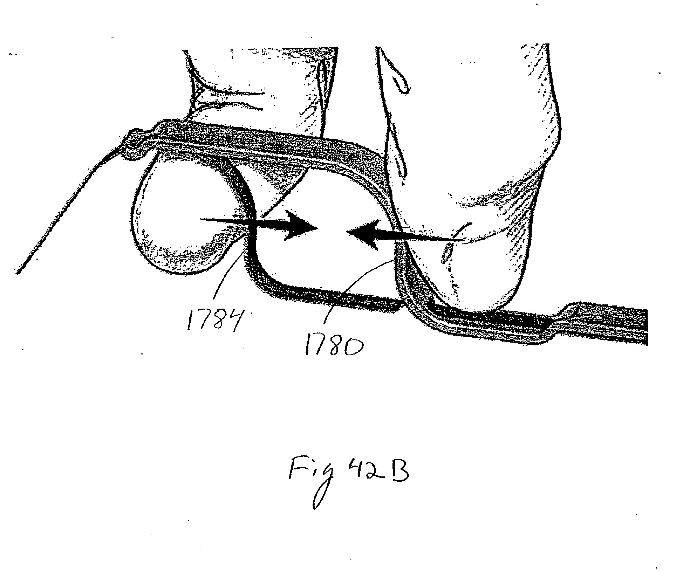

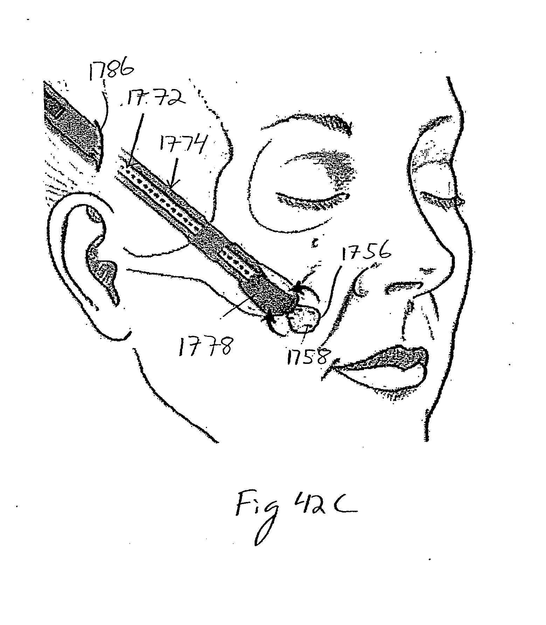

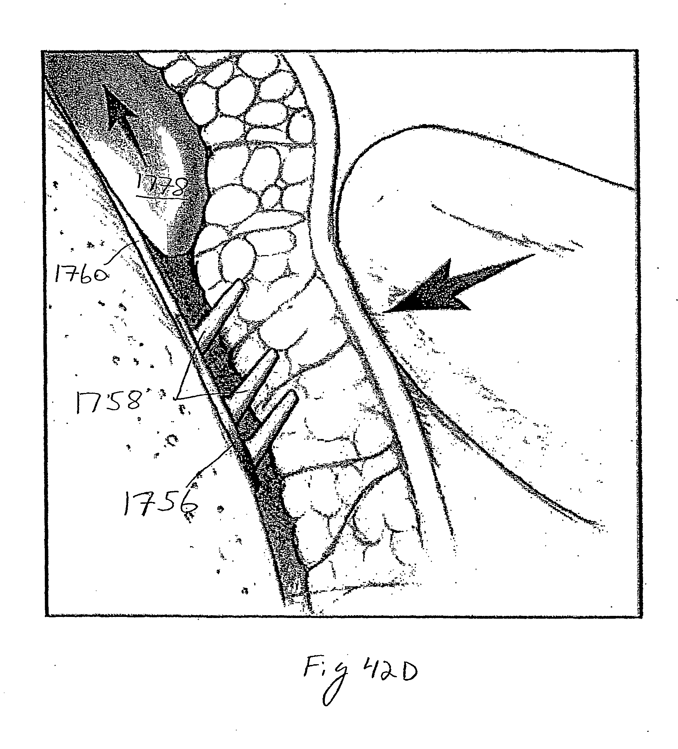

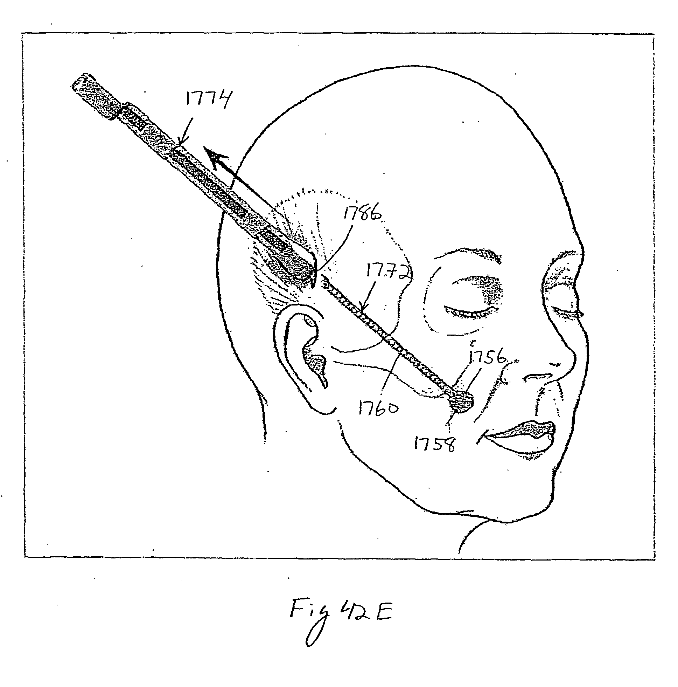

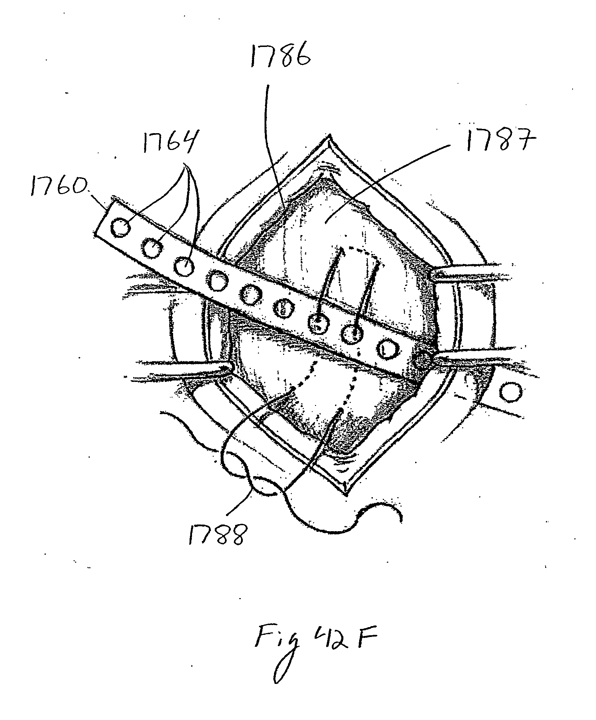

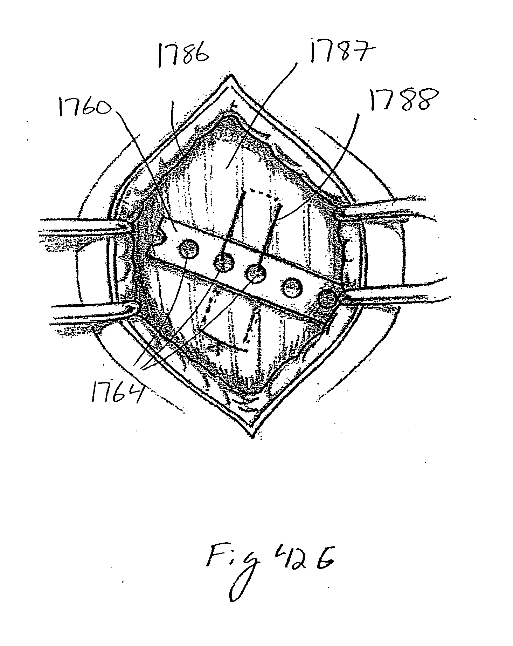

FIGS. 42A-42G illustrate the implementation of tissue approximation device (1772) for mid-face tissue approximation, utilizing the delivery device (1774). As illustrated in FIG. 42A, delivery device (1774) containing approximation device (1772) is inserted through a temporal incision (1786) over the temporal fascia (1787) until the backing/tines (1756/1758) (inside cup 1778) are disposed adjacent the tissue to be approximated. Protective cup (1778) prevents the tines (1758) from engaging any tissue during such insertion. Handles (1780/1784) are then pressed together, as shown in FIG. 42B, to force backing/tines (1756/1758) from cup (1778), as shown in FIG. 42C. Prior to and during removal of delivery device (1774) through the temporal incision (1786), pressure is exerted against the tissue over tines (1758) to set (i.e. affix) the tissue on device (1772) (so that the tines penetrate into and engage with the tissue), as shown in FIG. 42D. Once the delivery device (1774) is completely removed (see FIG. 42E), the leash (1760) is pulled to approximate the tissue engaged with tines (1758) into its desired position. At this point, suture (1788) is then used to secure the leash (1760) to the temporal fascia (1787) using one or more of the engagement holes (1764), as illustrated in FIG. 42F. Lastly, any excess amount of leash (1760) beyond the engagement holes (1764) used to secure the device (1772) in place can be severed or cut, as shown in FIG. 42G.

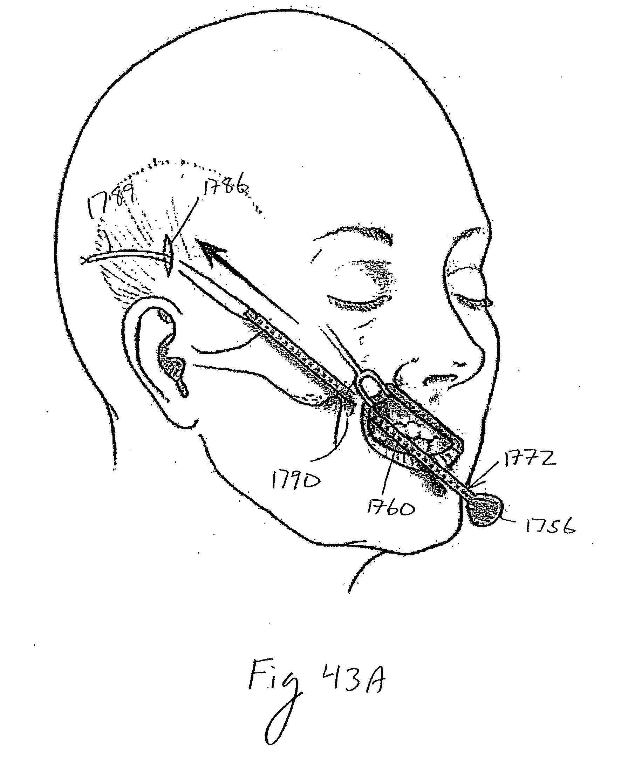

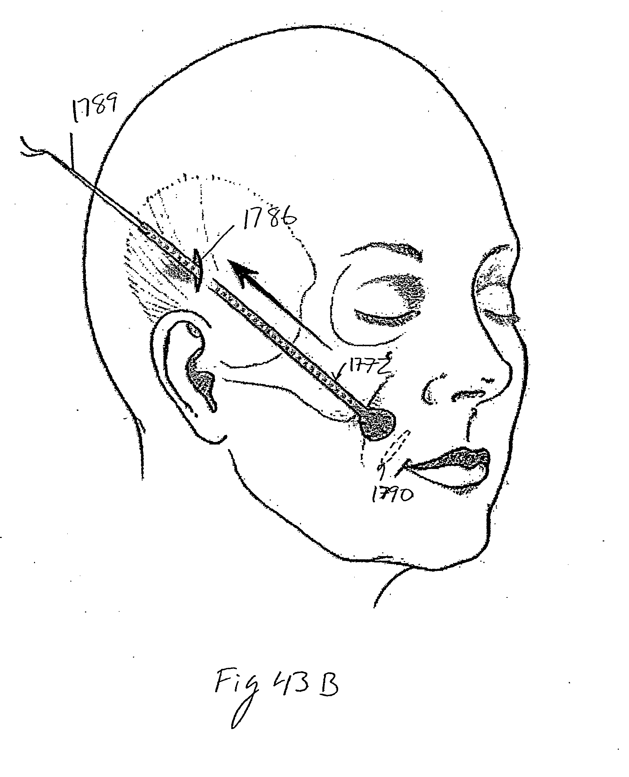

While delivery device (1774) is an ideal tool for inserting the approximation device (1772) in a manner where tines (1758) do not inadvertently and prematurely engage with tissue, it may be possible to insert approximation device (1772) without using delivery device (1774) through temporal incision (1786). Alternately, a suture (1789) extending through temporal incision (1786) can be used to draw approximation device (1772) up through a buccal incision (1790) below the tissue to be approximated, as shown in FIGS. 43A and 43B.

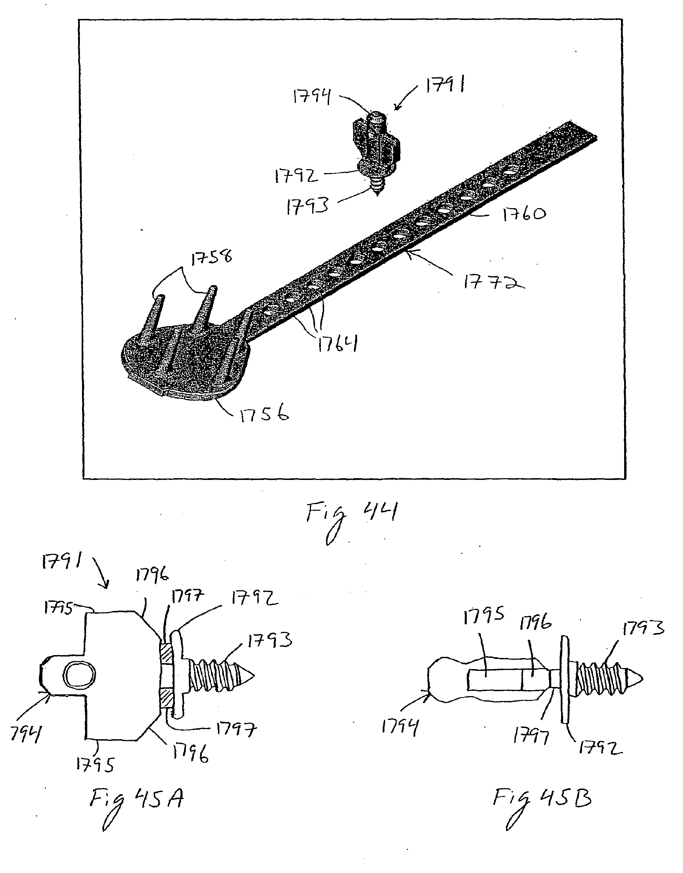

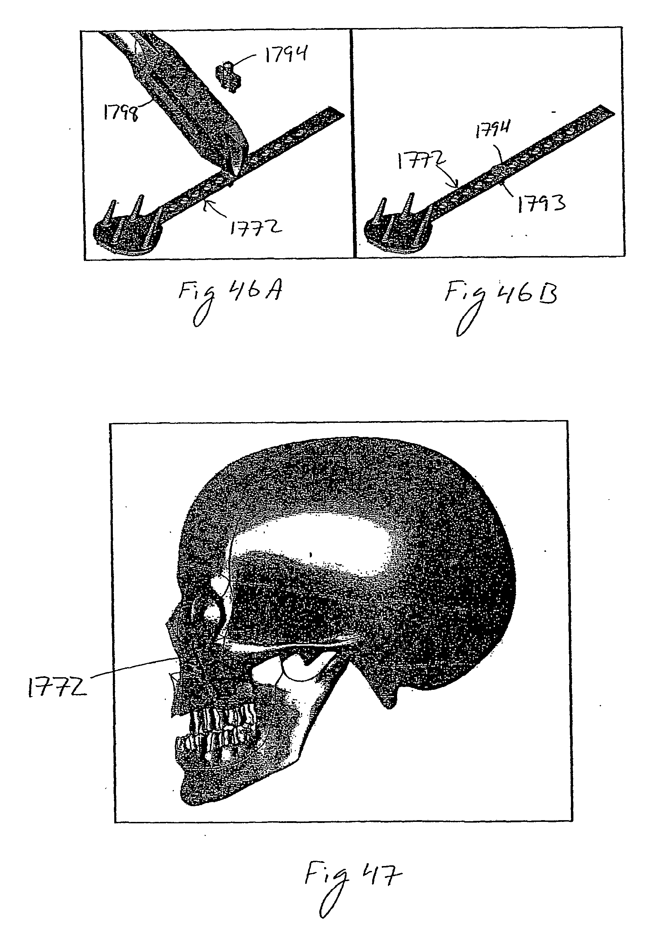

It should be noted that other tissue and/or bone attachment devices, such as posts, staples, screws, clips, etc., can be used instead of, or in addition to, suture (1788), to secure the device (1772) to the temporal fascia (1787), the underlying bone or skull, or any other tissue or bone. For example, FIGS. 44 and 45A/B illustrates a specially designed low profile medical screw (1791), which includes a flange (1792), a threaded shaft (1793) extending from one side of the flange (1792), and a screw head (1794) extending from the other side of the flange (1792). The screw head (1794) is configured to allow a rotation tool to engage with, to retain engagement with, and to rotate the screw head (1794) (for example, wings (1795) with sloped surfaces (1796) tapering down to a narrow neck portion (1797) that is attached to or formed with flange (1792)). The screw head (1791) is designed to enable a cutting tool (1798) to accurately engage with and cut off the screw head (1794) (i.e. cut through the neck portion (1797) where it meets flange (1792)), leaving a planar flange surface that is almost flush with the leash (1760), as illustrated in Figs. 46A and 46B. In the preferred embodiment, neck portion (1797) comprises a pair of narrow posts extending between the flange (1792) and the winged portion (1795) of head (1794), where the sloped portions (1796) of wings (1795) guide the cutting device to the neck portion (1797). The extremely low profile of screw (1791) allows implementation of the tissue approximation device (1772) in locations where the overlying tissue is quite thin, such as the tissue just below the eye as shown in FIG. 47.

It should also be noted that engagement holes (1764) need not be round as shown in the figures, but could be elongated, or even formed as perforations or thin slits, through which the suture can be passed by itself or via the aid of a needle. Alternately, engagement holes (1764) can be omitted altogether, and instead leash (1760) can have a thickness thin enough so that a needle carrying suture (1778) can be passed through the leash material, and therefore any location along leash (1760) can be selected for suture attachment.

Lastly, it should be noted that elongated support member (1776) need not necessarily terminate at the one end in protective cup (1778), but can terminate with any protective member (e.g. cup, rail, post, ring, tab, etc.) that lifts the tissue away from the tines (1758).

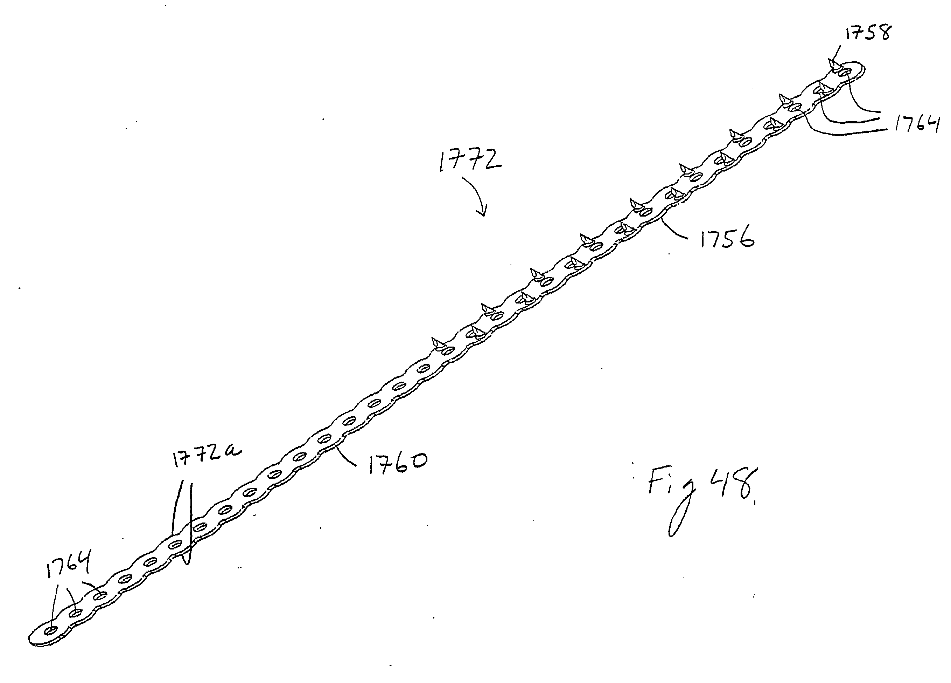

FIG. 48 illustrates an alternate configuration of approximation device (1772), where backing (1756) and leash (1760) are integrally formed together as a unitary elongated band, preferably but not necessarily having a uniform width and height. The leash portion (1760) contains the plurality of through-holes (1764) as described above. However, the backing portion (1756) contains both the tines (1758) and through-holes (1764). The through-holes (1764) preferably, but not necessarily, are linearly oriented along the length of approximation device (1772), including in an intermixed manner with the tines (1758) in the backing portion (1756). Through-holes (1764) not only serve as possible anchor points, but also allow for tissue ingrowth. Tissue fixation and in-growth can be enhanced by staggering of the tines on alternating sides of the through-holes as shown in Fig. 48. The side edges (1772a) of the device (1772) are preferably non-linear (e.g. scalloped or wavy), which has been found to drastically reduce the ability to see and feel the approximation device after implementation.

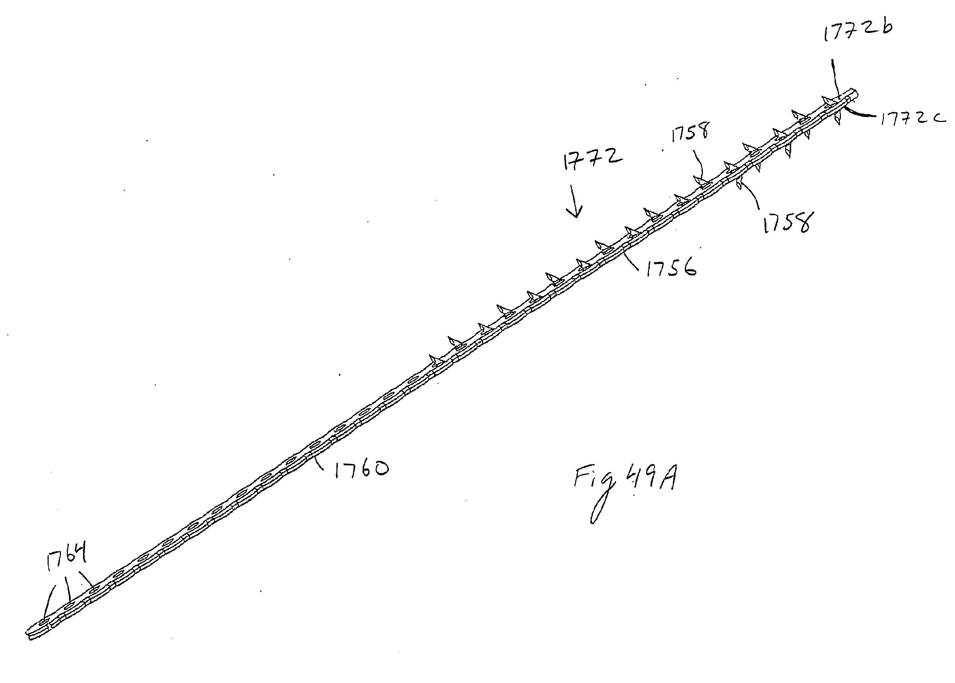

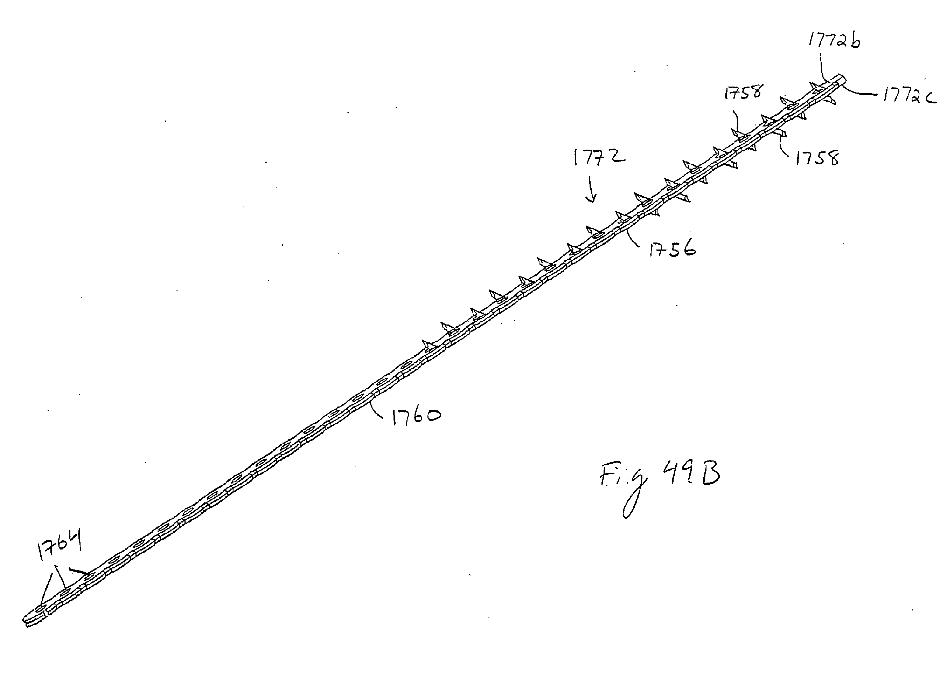

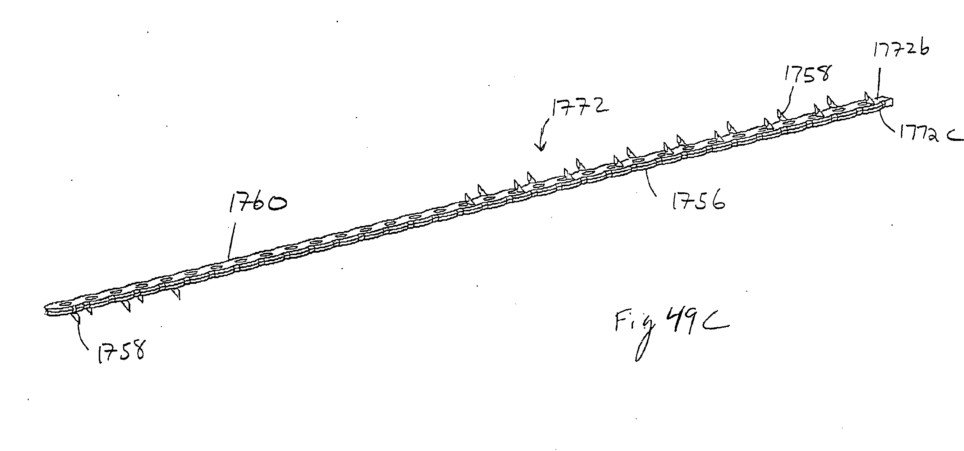

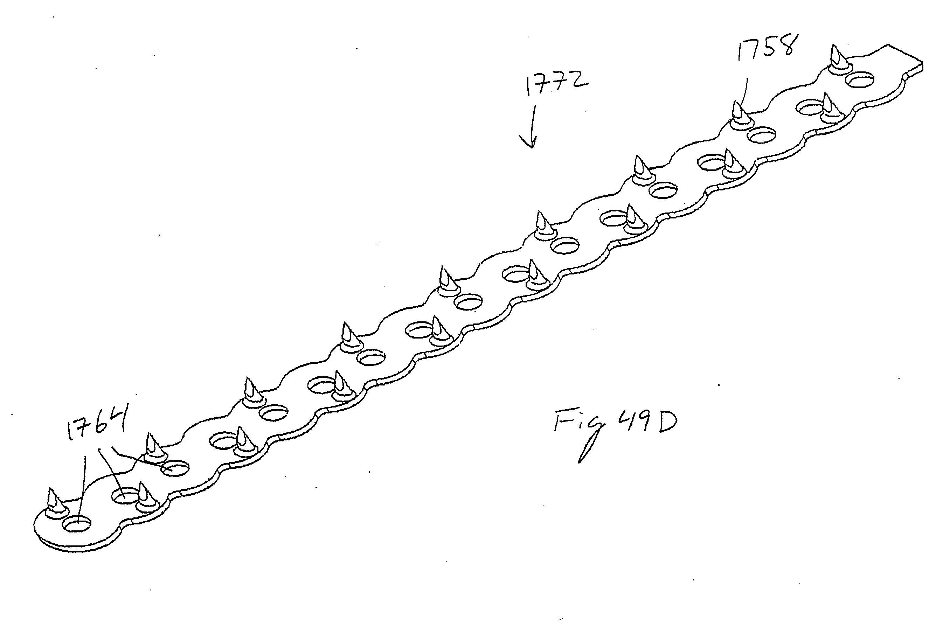

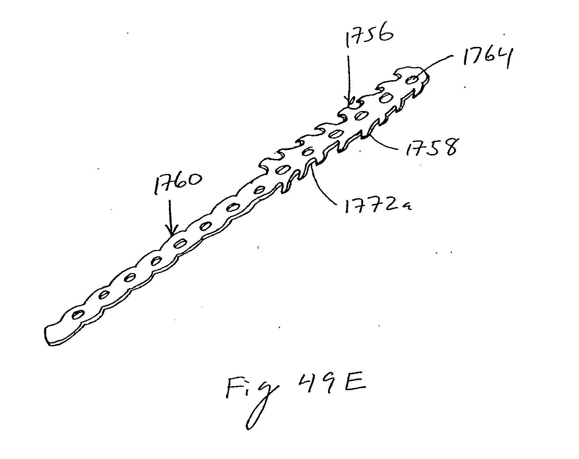

By having both the backing portion (1756) and the leash portion (1760) integrally formed together in a linearly extending manner as shown and described above, each of these portions can be individually cut to size for optimal implantation results, even after partial or complete implantation. For example, the length of fixation backing and thus the number of tines used for tissue fixation, and/or the length of leash portion used for extending the anchor positions to the anchor points, can be reduced in a customized manner by cutting either or both of the backing and leash portions (1756/1760) before, during or even after implantation of the device. Other variations of this embodiment include: forming tines (1758) on both the front (1772b) and back (1772c) sides of the backing portion (1756) all inclined toward the leash portion as shown in Fig. 49A (for fixating tissue on both sides of the approximation device in the same direction) or inclined in opposing directions as shown in Fig. 49B (for providing fixation or additional anchoring on both sides but in opposite directions), additionally forming tines (1758) on the front and/or back side of the leash portion (1760) as shown in Fig. 49C (for engaging tissue near or even on the other side of the anchor point), continuously forming the tines (1758) along both the backing and leash portions (1756/1760) as shown in Fig. 49D (for tissue fixation continuously along the entire length of the approximation device), and forming tines extending from the side edges 1772a and through-holes 1764 extending between front and back sides (1772b/1772c) as shown in Fig. 49E. Any combination of tines on the front and/or back sides of backing portion (1756), and/or tines on the front and/or back sides of leash portion (1760), and/or tines on side edges 1772a of backing and/or leash portions (1756/1760), are contemplated by the present invention.

The present invention also encompasses systems comprising any combination of spacers, fasteners, and supportive backings with tines which are useful for fracture fixation and soft tissue suspension.

Further, the present invention is not limited to bone fracture repair sites. The present invention may also be used in applications where no bone fractures are present or where bone fractures have healed. For example, the device may be attached to a healthy bone site to cure or compensate for sagging tissue. Moreover, the device of the present invention may be used to supplement previous surgeries in which the soft tissue was elevated from the underlying bone needs re-anchoring.

It is to be understood that the present invention is not limited to the embodiment(s) described above and illustrated herein, but encompasses any and all variations falling within the scope of the appended claims. For example, materials, processes and numerical examples described above are exemplary only, and should not be deemed to limit the claims.

## Claims

1. A tissue approximation assembly comprising an implantable tissue approximation device (1708, 1754, 1772) attachable to supporting tissue or bone, comprising:
a supportive backing (1756) having front and back sides (1772b, 1772c) and longitudinal side edges (1772a);
a plurality of attachment points (1730, 1758) extending from at least one of the front side (1772b) and back side (1772c) of the backing (1756); and
an elongated member (1760) extending from the backing (1756), wherein the elongated member (1760) is free of attachment points (1730, 1758), and wherein the elongated member (1760) includes a plurality of through-holes (1764) each formed through the elongated member (1760) at one of a plurality of locations along the elongated member (1760); **characterised in that** the tissue approximation assembly further comprises a delivery device (1774) engagable with the elongated member (1760), wherein the delivery device (1760) includes:
an elongated support member (1776) slidably attachable to the elongated member (1760), wherein the elongated support member (1776) terminates in a protective member (1778) for placement over the attachment points (1730,1758).

2. The assembly of claim 1, wherein the elongated member (1760) extends away from the backing (1756), and wherein the through-holes (1764) are formed along a single straight line extending along the elongated member (1760).

3. The assembly of claim 1 or 2, wherein the backing front and back sides (1772b, 1772c) are planar surfaces.

4. The assembly of any one of the preceding claims, wherein the elongated member (1760) includes front and back sides (1772b,1772c) through which the through-holes (1764) are formed.

5. The assembly of claim 4, wherein the backing front and back sides (1772b,1772c) and the elongated member front and back sides are integrally formed together in a co-planar manner.

6. The assembly of any one of the preceding claims, wherein the attachment points (1730,1758) extend from the backing (1756) in a non-orthogonal manner, with the attachment points (1730,1758) angled toward the elongated member (1760).

7. The assembly of any one of the preceding claims, wherein the delivery device (1774) further comprises:
an implementation member (1782) slidably attached to the elongated support member (1776) for pushing on at least one of the elongated member (1760) and the backing (1756) so that the elongated member (1760) slides relative to the elongated support member (1776) and so that the attachment points (1730,1758) are moved away from the protective member (1778).

8. The assembly of claim 7, wherein elongated support member (1776) includes a first handle (1780) and the implementation member (1782) includes a second handle (1784), and wherein the implementation member (1782) slides relative to the elongated member (1776) as the first and second handles (1780,1784) are squeezed toward each other.

9. The assembly of any one of the preceding claims, further comprising a medical screw (1791) for insertion through one of the through-holes (1764) to attach the elongated member (1776) to support tissue or bone, wherein the medical screw (1791) comprises:
a flange (1792) having first and second sides;
a threaded shaft (1793) extending from the first flange side;
a screw head (1794) extending from the second flange side, wherein the screw head (1794) is configured for grasping and turning the medical screw (1791) and is attached to the second flange via a narrow neck portion.

10. The assembly of claim 9, wherein the screw head (1794) includes a tapered portion adjacent the narrow neck portion (1797).

11. The assembly of claim 9, wherein the screw head (1794) includes a pair of wings (1795) having tapered portions adjacent the narrow neck portion (1797).

12. The assembly of any one of the preceding claims, wherein the elongated member (1760) and the supportive backing (1756) are integrally formed as a unitary elongated band.

13. The assembly of claim 12, wherein the unitary band has a substantially uniform width and thickness.

14. The assembly of claim 12, wherein the unitary band includes longitudinal side edges (1772a) that are wavy.

15. The assembly of claim 12, wherein the unitary band is rectangular in shape.

16. The assembly of any one of the preceding claims, wherein the supportive backing (1756) includes a plurality of through-holes (1764) formed through the supportive backing (1756) and intermixed among the plurality of attachment points (1730,1758).

17. The assembly of claim 16, wherein the through-holes (1764) of the supportive backing (1756) and the elongated member (1760) are oriented in a substantially straight line.

18. The assembly of claim 16, wherein the attachment points (1730,1758) of the supportive backing (1756) are oriented on alternating sides of the through-holes (1764) formed through the supportive backing (1756).

19. The assembly of any one of the preceding claims, wherein some of the plurality of attachment points (1730,1758) extend from the front side and others of the plurality of attachment points (1730,1758) extend from the back side (1772c).

20. The assembly of claim 19, wherein the attachment points (1730,1758) extend from the backing (1756) in a non-orthogonal manner, with the attachment points (1730,1758) extending from the front side (1772b) being angled toward the elongated member (1760) and the attachment points (1730,1758) extending from the back side (1772c) being angled away from the elongated member (1760).

21. The assembly of claim any one of claims 1 to 18, wherein that attachment points (1730,1758) extend from the front side (1772b) of the backing (1756), and wherein the back side (1772c) of the backing (1756) is free of attachment points.

22. The assembly of any one of the preceding claims, wherein the attachment points (1758) are arranged in a pentagonal configuration.

## Patentansprüche

1. Gewebeannährungsanordnung, umfassend eine implantierbare Gewebeannährungsvorrichtung (1708, 1754, 1772), die an einem stützenden Gewebe oder Knochen befestigt werden kann, umfassend:
einen unterstützenden Träger (1756) mit einer Vorderseite und einer Rückseite (1772b, 1772c) und mit Längsseitenkanten (1772a);
eine Vielzahl von Befestigungspunkten (1730, 1758), die sich zumindest von einer der Vorderseite (1772b) und Rückseite (1772c) des Trägers (1756) erstrecken; und
ein längliches Element (1760), das sich von dem Träger (1756) erstreckt,
wobei das längliche Element (1760) frei von Befestigungspunkten (1730, 1758) ist und wobei das längliche Element (1760) eine Vielzahl von Durchgangsöffnungen (1764) aufweist, die jeweils an einer der Vielzahl von Stellen entlang des länglichen Elements (1760) durch das längliche Element (1760) hindurch gebildet sind, **dadurch gekennzeichnet, dass** die Gewebeannäherungsanordnung ferner eine Abgabevorrichtung (1774) umfasst, die mit dem länglichen Element (1760) in Eingriff gebracht werden kann, wobei die Abgabevorrichtung (1760) umfasst:
ein längliches Stützelement (1776), das an dem länglichen Element (1760) verschiebbar befestigt werden kann, wobei das längliche Stützelement (1776) in einem Schutzelement (1778) zur Anordnung über den Befestigungspunkten (1730, 1758) endet.

2. Anordnung nach Anspruch 1, wobei sich das längliche Element (1760) von dem Träger (1756) weg erstreckt und wobei die Durchgangsöffnungen (1764) entlang einer einzigen geraden Linie gebildet sind, die sich entlang des länglichen Elements (1760) erstreckt.

3. Anordnung nach Anspruch 1 oder 2, wobei die Vorderseite und Rückseite (1772b, 1772c) des Trägers ebene Flächen sind.

4. Anordnung nach einem der vorhergehenden Ansprüche, wobei das längliche Element (1760) eine Vorderseite und eine Rückseite (1772b, 1772c) hat, durch welche hindurch die Durchgangsöffnungen (1764) gebildet sind.

5. Anordnung nach Anspruch 4, wobei die Vorderseite und die Rückseite (1772b, 1772c) des Trägers und die Vorderseite und die Rückseite des länglichen Elements einteilig in einer Ebene liegend ausgebildet sind.

6. Anordnung nach einem der vorhergehenden Ansprüche, wobei sich die Befestigungspunkte (1730, 1758) nichtorthogonal von dem Träger (1756) erstrecken, wobei die Befestigungspunkte (1730, 1758) in Richtung auf das längliche Element (1760) abgewinkelt sind.

7. Anordnung nach einem der vorhergehenden Ansprüche, wobei die Abgabevorrichtung (1774) ferner umfasst:
ein Implementierungselement (1782), das an dem länglichen Stützelement (1776) verschiebbar befestigt ist, um zumindest das längliche Element (1760) oder zumindest den Träger (1756) derart anzuschieben, dass das längliche Element (1760) relativ zu dem länglichen Stützelement (1776) gleitet, und derart, dass die Befestigungspunkte (1730, 1758) von dem Schutzelement (1778) weg bewegt werden.

8. Anordnung nach Anspruch 7, wobei das längliche Stützelement (1776) einen ersten Griff (1780) und das Implementierungselement (1782) einen zweiten Griff (1784) aufweist und wobei das Implementierungselement (1782) relativ zu dem länglichen Element (1776) gleitet, wenn der erste und der zweite Griff (1780, 1784) in Richtung zueinander gedrückt werden.

9. Anordnung nach einem der vorhergehenden Ansprüche, ferner umfassend eine medizinische Schraube (1791) zum Einsetzen durch eine der Durchgangsöffnungen (1776), um das längliche Element (1776) an dem stützenden Gewebe oder Knochen zu befestigen, wobei die medizinische Schraube (1791) umfasst:
einen Flansch (1792) mit einer ersten und einer zweiten Seite;
einen Gewindeschaft (1793), der sich von der ersten Flanschseite erstreckt;
einen Schraubenkopf (1794), der sich von der zweiten Flanschseite erstreckt, wobei der Schraubenkopf (1794) für das Fassen und Drehen der medizinischen Schraube (1791) konfiguriert ist und über einen schmalen Halsabschnitt an dem zweiten Flansch befestigt ist.

10. Anordnung nach Anspruch 9, wobei der Schraubenkopf (1794) einen an den schmalen Halsabschnitt (1797) angrenzenden verjüngten Abschnitt aufweist.

11. Anordnung nach Anspruch 9, wobei der Schraubenkopf (1794) ein Paar Flügel (1795) aufweist, die angrenzend an den schmalen Halsabschnitt verjüngte Bereiche haben.

12. Anordnung nach einem der vorhergehenden Ansprüche, wobei das längliche Element (1760) und der unterstützende Träger (1756) einteilig als unitäres längliches Band ausgebildet sind.

13. Anordnung nach Anspruch 12, wobei das unitäre Band eine im Wesentlichen einheitliche Breite und Dicke hat.

14. Anordnung nach Anspruch 12, wobei das unitäre Band Längsseitenkanten (1772a) aufweist, die wellig sind.

15. Anordnung nach Anspruch 12, wobei das unitäre Band in der Form rechteckig ist.

16. Anordnung nach einem der vorhergehenden Ansprüche, wobei der unterstützende Träger (1756) eine Vielzahl von Durchgangsöffnungen (1764) aufweist, die durch den unterstützenden Träger (1756) hindurch gebildet sind und die unter die Vielzahl von Befestigungspunkten (1730, 1758) gemischt sind.

17. Anordnung nach Anspruch 16, wobei die Durchgangsöffnungen (1754) des unterstützenden Trägers (1756) und des länglichen Elements (1760) im Wesentlichen in einer geraden Linie orientiert sind.

18. Anordnung nach Anspruch 16, wobei die Befestigungspunkte (1730, 1758) des unterstützenden Trägers (1756) auf abwechselnden Seiten der durch den unterstützenden Träger (1756) hindurch gebildeten Durchgangsöffnungen (1764) orientiert sind.

19. Anordnung nach einem der vorhergehenden Ansprüche, wobei sich einige der Vielzahl von Befestigungspunkten (1730, 1758) von der Vorderseite und andere der Vielzahl von Befestigungspunkten (1730, 1758) von der Rückseite (1772c) erstrecken.

20. Anordnung nach Anspruch 19, bei der sich die Befestigungspunkte (1730, 1758) nichtorthogonal von dem Träger (1756) erstrecken, wobei die sich von der Vorderseite (1772b) erstreckenden Befestigungspunkte (1730, 1758) in Richtung auf das längliche Element (1760) abgewinkelt sind und die sich von der Rückseite (1772c) erstreckenden Befestigungspunkte (1730, 1758) von dem länglichen Element (1760) weg abgewinkelt sind.

21. Anordnung nach einem der Ansprüche 1 bis 18, wobei sich die Befestigungspunkte (1730, 1758) von der Vorderseite (1772b) des Trägers (1756) erstrecken und wobei die Rückseite (1772c) des Trägers (1756) frei von Befestigungspunkten ist.

22. Anordnung nach einem der vorhergehenden Ansprüche, wobei die Befestigungspunkte (1758) in einer pentagonalen Konfiguration angeordnet sind.

## Revendications

1. Ensemble de rapprochement de tissus comprenant un dispositif de rapprochement de tissus implantable (1708, 1754, 1772) pouvant être fixé à un tissu ou os de support, comprenant :
un élément de support (1756) ayant des côtés avant et arrière (1772b, 1772c) et des bords latéraux longitudinaux (1772a) ;
une pluralité de points d'attache (1730, 1758) s'étendant d'au moins un parmi le côté avant (1772b) et le côté arrière (1772c) du support (1756) ; et
un organe allongé (1760) s'étendant depuis de support (1756), dans lequel l'organe allongé (1760) n'a pas de point d'attache (1730, 1758), et dans lequel l'organe allongé (1760) comporte une pluralité de trous débouchants (1764) formés chacun à travers l'organe allongé (1760) à une de la pluralité de positions le long de l'organe allongé (1760) ; **caractérisé en ce que** l'ensemble de rapprochement de tissus comprend en outre un dispositif d'administration (1774) pouvant être mis en prise avec l'organe allongé (1760), dans lequel le dispositif d'administration (1760) comprend :
un organe de support allongé (1776) fixé de façon coulissante à l'organe allongé (1760), dans lequel l'organe de support allongé (1776) se termine en un organe protecteur (1778) destiné à être placé au-dessus des points d'attache (1730, 1758).

2. Ensemble selon la revendication 1, dans lequel l'organe allongé (1760) s'étend en s'éloignant du support (1756), et dans lequel les trous débouchants (1764) sont formés le long d'une seule ligne droite s'étendant le long de l'organe allongé (1760).

3. Ensemble selon la revendication 1 ou 2, dans lequel les côtés avant et arrière du support (1772b, 1772c) sont des surfaces planes.

4. Ensemble selon l'une quelconque des revendications précédentes, dans lequel l'organe allongé (1760) comporte des côtés avant et arrière (1772b, 1772c) à travers lesquelles les trous débouchants (1764) sont formés.

5. Ensemble selon la revendication 4, dans lequel les côtés avant et arrière du support (1772b, 1772c) et les côtés avant et arrière de l'organe allongé sont solidairement formés de manière coplanaire.

6. Ensemble selon l'une quelconque des revendications précédentes, dans lequel les points d'attache (1730, 1758) s'étendent depuis le support (1756) de manière orthogonale, avec les points d'attache (1730, 1758) orientés vers l'organe allongé (1760).

7. Ensemble selon l'une quelconque des revendications précédentes, dans lequel le dispositif d'administration (1774) comprend en outre :
un organe d'exécution (1782) fixé de façon coulissante à partir du support allongé (1776) pour pousser sur au moins un parmi les membres allongées (1760) et le support (1756) afin que l'organe allongé (1760) coulisse par rapport à l'organe de support allongé (1776) et afin que les points d'attache (1730, 1758) soient éloignés de l'organe protecteur (1778).

8. Ensemble selon la revendication 7, dans lequel l'organe de support allongé (1776) comporte une première poignée (1780) et l'organe d'exécution (1782) comporte une deuxième poignée (1784), et dans lequel l'organe d'exécution (1782) coulisse par rapport à l'organe allongé (1776) lorsque les première et deuxième poignées (1780, 1784) sont pressées l'une vers l'autre.

9. Ensemble selon l'une quelconque des revendications précédentes, comprenant en outre une vis médicale (1791) destinée à être insérée à travers l'un des trous débouchants (1764) pour fixer l'organe allongé (1776) au tissu ou os de support, dans lequel ladite vis médicale (1791) comprend :
un manchon (1792) ayant des premier et deuxième côtés ;
un arbre fileté (1793) s'étendant depuis le premier côté du manchon ;
une tête de vis (1794) s'étendant depuis le deuxième côté du manchon, dans lequel la tête de vis (1794) est configurée pour saisir et tourner la vis médicale (1791) et est fixée sur le deuxième manchon vie une partie étroite formant un cou.

10. Ensemble selon la revendication 9, dans lequel la tête de vis (1794) comporte une partie conique adjacente à la partie étroite formant un cou (1797).

11. Ensemble selon la revendication 9, dans lequel la tête de vis (1794) comporte une paire d'ailes (1795) ayant des parties coniques adjacentes à la partie étroite formant un cou (1797).

12. Ensemble selon l'une quelconque des revendications précédentes, dans lequel l'organe allongé (1760) et l'élément de support (1756) sont formés de façon solidaire en bande allongée unitaire.

13. Ensemble selon la revendication 12, dans lequel la bande unitaire a une largeur et une épaisseur qui sont sensiblement uniformes.

14. Ensemble selon la revendication 12, dans lequel la bande unitaire comporte des bords latéraux longitudinaux (1772a) qui sont ondulés.

15. Ensemble selon la revendication 12, dans lequel la bande unitaire est de forme rectangulaire.

16. Ensemble selon l'une quelconque des revendications précédentes, dans lequel l'élément de support (1756) comporte une pluralité de trous débouchants (1764) formés à travers l'élément de support (1756) et entremêlés au sein d'une pluralité de points d'attache (1730, 1758).

17. Ensemble selon la revendication 16, dans lequel les trous débouchants (1764) de l'élément de support (1756) et l'organe allongé (1760) sont orientés vers une ligne sensiblement droite.

18. Ensemble selon la revendication 16, dans lequel les points d'attache (1730, 1758) de l'élément de support (1756) sont orientés vers des côtés alternants des trous débouchants (1764) formés à travers l'élément de support (1756).

19. Ensemble selon l'une quelconque des revendications précédentes, dans lequel certains de la pluralité de points d'attache (1730, 1758) s'étendent depuis le côté avant et les autres de la pluralité de points d'attache (1730, 1758) s'étendent depuis le côté arrière (1772c).

20. Ensemble selon la revendication 19, dans lequel les points d'attache (1730, 1758) s'étendent depuis le support (1756) de manière non-orthogonale, avec les points d'attache (1730, 1758) s'étendant depuis le côté avant (1772b) étant orientés vers l'organe allongé (1760) et les points d'attache (1730,758) s'étendant depuis le côté arrière (1772c) étant orientés en s'éloignant de l'organe allongé (1760).

21. Ensemble selon l'une quelconque des revendications 1 à 18, dans lequel les points d'attache (1730, 1758) s'étendent depuis le côté avant (1772b) du support (1756), et dans lequel le côté arrière (1772c) du support (1756) n'a pas de points d'attache.

22. Ensemble selon l'une quelconque des revendications précédentes, dans lequel les points d'attache (1758) sont disposés selon une configuration pentagonale.
